# EUROPEAN PATENT APPLICATION

(11) **EP 3 192 808 A1**
(43) Date of publication of application: **19.07.2017**
(21) Application number: 17159003.7
(22) Date of filing: 26.11.2008
(51) Int. Cl.: C07K 16/18, A61K 39/395, A61P 19/02, G01N 33/53, C07K 16/00, G01N 33/543

(54) **14-3-3 ANTAGONISTS FOR THE PREVENTION AND TREATMENT OF ARTHRITIS**

(30) Priority: 27.11.2007 US 990520 P; 30.06.2008 US 77123 P
(62) Divisional of application: 08853323.7
(71) Applicant: THE UNIVERSITY OF BRITISH COLUMBIA, Vancouver, British Columbia V6T 1Z3 (CA)
(72) Inventor: Marotta, Anthony, Burnaby, British Columbia V5C 5Y8 (CA); Ghahary, Aziz, Vancouver, British Columbia V6C 2C6 (CA); Maksymowych, Walter Wolodymyr Peter, Edmonton, Alberta T6G 2S2 (CA); Kilani, Ruhangiz, Vancouver, British Columbia V6C 2C6 (CA)
(74) Representative: van Kooij, Adriaan

(57) **Abstract**

Methods for treating arthritis comprising 14-3-3 antagonists that are capable of specifically binding to extracellularly localized 14-3-3 eta and/or 14-3-3 gamma protein isoforms are provided. In preferred embodiments, the 14-3-3 antagonist is an inhibitory peptide or an anti-14- 3-3 antibody. The 14-3-3 antagonists are also formulated in a pharmaceutical composition and used in a method for reducing matrix metalloprotease (MMP) expression in the synovial fluid of a patient, wherein the MMP is MMP-1 or MMP-3.

## Description

### CROSS-REFERENCE TO RELATED APPLICATIONS

This application claims the benefit of U.S. Provisional Patent Application Serial No. 60/990,520, filed 27 November 2007, and U.S. Provisional Patent Application Serial No. 61/077,123, filed 30 June 2008, which are expressly incorporated herein in their entirety by reference.

### FIELD OF THE INVENTION

The invention relates to the involvement of 14-3-3 proteins in arthritis, and compositions and methods for the prevention and treatment of arthritis.

### BACKGROUND OF THE INVENTION

Arthritis, or arthralgia, generally refers to inflammatory disorders of the joints of the body, and is usually accompanied by pain, swelling and stiffness. Arthritis may result from any of several causes including infection, trauma, degenerative disorders, metabolic disorders or disturbances or other unknown etiologies. Osteoarthritis (OA) is a common form of arthritis that may occur following trauma to a joint, following an infection of a joint or simply as a result of aging. Osteoarthritis is also known as degenerative joint disease. Rheumatoid arthritis (RA) is traditionally considered a chronic, inflammatory autoimmune disorder that causes the immune system to attack the joints. It is a disabling and painful inflammatory condition which can lead to substantial loss of mobility due to pain and joint destruction. Ankylosing spondylitis (AS) is a chronic, painful, degenerative inflammatory arthritis primarily affecting the spine and sacroiliac joints, causing eventual fusion of the spine.

The body's articulating joints are called synovial joints, and each synovial joint generally comprises the opposing ends of two adjacent bones. The ends of the bones are encased in cartilage tissue while the entire joint area is encased in a protective soft tissue called synovium which comprises synovial membrane. The synovial membrane produces and releases a lubricating synovial fluid into cavities within the joint. In normal joints, the volume of synovial fluid is quite small. In addition to its lubricating function, synovial fluid also acts as a reservoir for solutes and a few resting mononuclear and synovial cells.

The synovium can become irritated and thickened in response to many insults believed to promote arthritis, including trauma to the joint and/or malfunction of the body's immune system. The consequences of such insults include excessive production and release of synovial fluid into the joint, thereby causing swelling within and about the joint area. The increased volumes are typically accompanied by increased concentrations in the synovial fluid of fibroblast-like synoviocyte cells (FLS cells), pro-inflammatory cytokines such as interleukin-1 (IL-1) and tumor necrosis factor (TNF-alpha), histamine proteins and peptides, and degradative enzymes such as matrix metalloproteases (MMPs). The FLS cells comprise about two-thirds of the synovial cells in normal synovial fluid, have well-defined secretory systems, and under conditions of trauma or inflammation commonly secrete large amounts of MMPs into the synovial fluid, specifically MMP-1, 3, 8, 9, 10, 11 and 13. MMP-1 and MMP-3 are considered to have significant roles in the progressive structural damage of cartilage and underlying bone tissues comprising joints. Known factors that activate FLS cells to produce MMP-1 and MMP-3 include IL-1 and TNF-alpha.

The causative agents for RA, SA and OA are currently not well-defined. However, the physiological events associated with progression of the disease, from prolonged periods of swelling and inflammation caused by excessive synovial fluid accumulation in the joints, through degradation and deterioration of the cartilage and underlying bone tissues by degradative enzyme activities, and the accompanying FLS cell proliferation into bone which results in permanent structural damage, are known. If detected early enough, the potential long-term deleterious effects of disease can be reversed, or at least minimized, with appropriate physical and medical therapies. Accordingly, considerable efforts have been placed on the identification of suitable biomarkers for early identification of arthritis. To this end, Kilani et al. (2007, J. Rheum. 34: 1650-1657; WO 2007/128132) have reported that two members of the 14-3-3 protein family, particularly 14-3-3 eta and 14-3-3 gamma, are present within the synovial fluid and serum of patients with arthritis, and these isoforms are directly correlated with the levels of MMP-1 and MMP-3 in the synovial fluid and serum.

14-3-3 proteins are a family of conserved intracellular regulatory molecules that are ubiquitously expressed in eukaryotes. 14-3-3 proteins have the ability to bind a multitude of functionally diverse signaling proteins, including kinases, phosphatases, and transmembrane receptors. Indeed, more than 100 signaling proteins have been reported as 14-3-3 ligands. 14-3-3 proteins may be considered evolved members of the Tetratrico Peptide Repeat superfamily. They generally have 9 or 10 alpha helices, and usually form homo- and/or hetero-dimer interactions along their amino-termini helices. These proteins contain a number of known domains, including regions for divalent cation interaction, phosphorylation & acetylation, and proteolytic cleavage, among others. There are seven distinct genetically encoded isoforms of the 14-3-3 proteins that are known to be expressed in mammals, with each isoform comprising between 242-255 amino acids. The seven 14-3-3 protein isoforms are designated as 14-3-3 α/β (alpha/beta), 14-3-3 δ/ξ (delta/zeta), 14-3-3 ε (epsilon), 14-3-3 γ (gamma), 14-3-3 η (eta), 14-3-3 τ/□ (tau/theta), and 14-3-3 □ (sigma/stratifin).

### SUMMARY OF THE INVENTION

The invention stems in part from the findings that (i) 14-3-3 protein is aberrantly localized in the extracellular synovial space in arthritis, (ii) such extracellular 14-3-3 protein can induce effectors of arthritis, and (iii) 14-3-3 antagonists directed to such extracellular 14-3-3 proteins can reduce the effectors of arthritis.

In one aspect, the invention provides methods of treating arthritis. In a preferred embodiment, the invention provides methods of treating a disease selected from the group consisting of ankylosing spondylitis, Behçet's Disease, diffuse idiopathic skeletal hyperostosis (DISH), Ehlers-Danlos Syndrome (EDS), Felty's Syndrome, fibromyalgia, gout, infectious arthritis, juvenile arthritis, lupus, mixed connective tissue disease (MCTD), osteoarthritis, Paget's Disease, polymyalgia rheumatica, polymyositis and dermatomyositis, pseudogout, psoriatic arthritis, Raynaud's Phenomenon, reactive arthritis, rheumatoid arthritis, scleroderma, Sjögren's Syndrome, Still's Disease, and Wegener's granulomatosis.

The methods involve administration of a 14-3-3 antagonist to an affected patient, wherein the 14-3-3 antagonist is targeted to 14-3-3 protein that is localized extracellularly. In a preferred embodiment, the 14-3-3 protein is 14-3-3 eta or 14-3-3 gamma.

The 14-3-3 antagonists used may be prior art compositions, or novel compositions disclosed herein. Therapeutic compositions are formulated and administration is such that the 14-3-3 antagonist so delivered is available to engage extracellular 14-3-3 protein. In one embodiment, the 14-3-3 antagonist is a peptide or an anti-14-3-3 antibody.

In a preferred embodiment, the 14-3-3 antagonist used is capable of inhibiting the induction of MMP by a 14-3-3 protein to which it binds. Preferably, the MMP is selected from the group consisting of MMP-1, 3, 8, 9, 10, 11 and 13, with MMP-1 and MMP-3 being especially preferred.

In one embodiment, the method involves a combination treatment, wherein at least one other therapeutic agent is administered in addition to one or more 14-3-3 antagonists. In a preferred embodiment, the therapeutic agent is selected from the group consisting of disease-modifying antirheumatic drugs (DMARDs) and disease modifying osteoarthritis drugs (DMOADs; for example, see Loeser, Reumatologia, 21:104-106, 2005). In an especially preferred embodiment, one or more anti-14-3-3 antagonists is administered in combination with at least one agent selected from the group consisting of anti-TNFα antibody, anti-IL-1 antibody, anti-CD4 antibody, anti-CTLA4 antibody, anti-IL-6 antibody, anti-CD20 antibody, leflunomide, sulfasalazine, and methotrexate.

In one embodiment, a 14-3-3 antagonist is administered in the form of an encoding nucleic acid that is expressed to deliver the 14-3-3 antagonist.

In one embodiment, the method involves administering to a patient a cell that delivers a 14-3-3 antagonist. In a preferred embodiment, the 14-3-3 antagonist so delivered is a peptide or an anti-14-3-3 antibody. In a preferred embodiment, the cell is a fibroblast or an FLS cell.

In one aspect, the invention provides prophylactic methods for preventing the development of arthritis in a subject at risk of developing arthritis. The methods comprise administering to the subject one or more 14-3-3 antagonists.

In one aspect, the invention provides methods for reducing the damage to a joint injured by trauma. The methods comprise administering one or more 14-3-3 antagonists to a subject having a joint injured by trauma. In one embodiment, a 14-3-3 antagonist is administered as a component of a combination therapy described herein.

In one aspect, the invention provides methods of decreasing MMP expression. In one embodiment, the MMP expression to be decreased is in the synovium. The methods comprise delivering one or more 14-3-3 antagonists to a tissue or compartment in which MMP producing cells are present, wherein the MMP producing cells are responsive to 14-3-3 proteins to which the 14-3-3 antagonists bind. Delivery may be direct to the affected tissue or compartment, or indirect. In a preferred embodiment, the responsive cells are fibroblasts or FLS cells.

In a preferred embodiment, the MMP expression that is to be decreased is MMP expression that is associated with arthritis.

In a preferred embodiment, the MMP expression that is to be decreased is that of an MMP selected from the group consisting of MMP-1, 3, 8, 9, 10, 11 and 13. In an especially preferred embodiment, the MMP expression that is to be decreased is that of MMP-1 or MMP-3.

In one aspect, the invention provides methods of inhibiting MMP induction by a 14-3-3 protein. Inhibition may be partial or complete. The methods comprise delivering one or more 14-3-3 antagonists to a tissue or compartment in which MMP producing cells are present, wherein the MMP producing cells are responsive to 14-3-3 proteins to which the 14-3-3 antagonists specifically bind. Delivery may be direct to the affected tissue or compartment, or indirect. In a preferred embodiment, the one or more 14-3-3 antagonists are administered to the synovium. In a preferred embodiment, the responsive cells are fibroblasts or FLS cells.

In a preferred embodiment, the MMP induction that is to be inhibited is that of an MMP which is upregulated in arthritis.

In a preferred embodiment, the MMP induction that is to be inhibited is that of an MMP selected from the group consisting of MMP-1, 3, 8, 9, 10, 11 and 13. In an especially preferred embodiment, the MMP induction that is to be inhibited is that of MMP-1 or MMP-3.

In one aspect, the invention provides methods of decreasing joint swelling in a subject. The methods comprise administering one or more 14-3-3 antagonists to an affected subject.

In one aspect, the invention provides methods of decreasing cartilage degradation in a subject. The methods comprise administering one or more 14-3-3 antagonists to an affected subject.

In one aspect, the invention provides methods of decreasing bone degradation in a subject. The methods comprise administering one or more 14-3-3 antagonists to an affected subject.

In one aspect, the invention provides methods of decreasing pro-inflammatory cytokine accumulation in synovial fluid. The methods comprise administering one or more 14-3-3 antagonists to an affected subject.

For methods involving administration of a 14-3-3 antagonist to an affected subject, in a preferred embodiment, intracapsular delivery of antagonist is used. In another embodiment, systemic delivery of antagonist is used. The therapeutic compositions are formulated and administration is such that the 14-3-3 antagonist so delivered is available to engage extracellularly localized 14-3-3 protein.

In one embodiment, the 14-3-3 antagonist is a peptide. In a preferred embodiment, the peptide comprises the amino acid sequence designated "R-18". In another preferred embodiment, the peptide consists essentially of the R-18 sequence. In one embodiment, the peptide comprises multiple iterations of the R-18 sequence. In another preferred embodiment, the peptide binds to a region of 14-3-3 protein that is capable of binding to R-18. In another preferred embodiment, the peptide binds to a region of a 14-3-3 protein that is capable of binding to an intracellular 14-3-3 binding partner, preferably Raf. In one embodiment, the peptide binds to a 14-3-3 protein without disrupting binding of the 14-3-3 protein to an intracellular 14-3-3 binding partner.

In one embodiment, the 14-3-3 antagonist is a phosphopeptide.

In one embodiment, the 14-3-3 antagonist is a mode I phosphopeptide, as is known in the art.

In another embodiment, the 14-3-3 antagonist is a mode II phosphopeptide, as is known in the art.

In one embodiment, the 14-3-3 antagonist is an anti-14-3-3 antibody. In one embodiment, the anti-14-3-3 antibody is a pan 14-3-3 antibody. In another embodiment, the anti-14-3-3 antibody is capable of distinguishing between 14-3-3 isoforms. In a preferred embodiment, the anti-14-3-3 antibody specifically binds to a peptide selected from the group consisting of 14-3-3 loop peptides, 14-3-3 helix peptides, and non-helix 14-3-3 peptides.

In one embodiment, the anti-14-3-3 antibody is an anti-14-3-3 gamma antibody. In a preferred embodiment, the anti-14-3-3 gamma antibody binds to a 14-3-3 peptide comprising a segment of the amino acid sequence set forth in SEQ ID NO:64. In a preferred embodiment, the segment is at least 6, more preferably at least 7, more preferably at least 8 amino acids in length.

In a preferred embodiment, an anti-14-3-3 gamma antibody binds to a 14-3-3 peptide that is a 14-3-3 gamma loop peptide. In a preferred embodiment, the 14-3-3 gamma loop peptide comprises an amino acid sequence selected from the group consisting of SEQ ID NOs:44-49. In another embodiment, the 14-3-3 gamma loop peptide consists essentially of an amino acid sequence selected from the group consisting of SEQ ID NOs:44-49. In another embodiment, an anti-14-3-3 gamma antibody binds to a region of 14-3-3 gamma that overlaps with an amino acid sequence corresponding to a sequence selected from the group consisting of SEQ ID NOs:44-49.

In another preferred embodiment, an anti-14-3-3 gamma antibody binds to a 14-3-3 peptide that is a 14-3-3 gamma helix peptide. In a preferred embodiment, the 14-3-3 gamma helix peptide comprises an amino acid sequence selected from the group consisting of SEQ ID NOs:33-43. In another embodiment, the 14-3-3 gamma helix peptide consists essentially of an amino acid sequence selected from the group consisting of SEQ ID NOs:33-43. In another embodiment, an anti-14-3-3 gamma antibody binds to a region of 14-3-3 gamma that overlaps with an amino acid sequence corresponding to a sequence selected from the group consisting of SEQ ID NOs:33-43.

In another preferred embodiment, an anti-14-3-3 gamma antibody binds to a 14-3-3 peptide that is a non-helix 14-3-3 gamma peptide. In a preferred embodiment, the non-helix 14-3-3 gamma peptide comprises an amino acid sequence selected from the group consisting of SEQ ID NOs:50-62. In another embodiment, the non-helix 14-3-3 gamma peptide consists essentially of an amino acid sequence selected from the group consisting of SEQ ID NOs:50-62. In another embodiment, an anti-14-3-3 gamma antibody binds to a region of 14-3-3 gamma that overlaps with an amino acid sequence corresponding to a sequence selected from the group consisting of SEQ ID NOs:50-62.

In one embodiment, the anti-14-3-3 antibody is an anti-14-3-3 eta antibody. In a preferred embodiment, the anti-14-3-3 eta antibody binds to a 14-3-3 peptide comprising a segment of the amino acid sequence set forth in SEQ ID NO:63. In a preferred embodiment, the segment is at least 6, more preferably at least 7, more preferably at least 8 amino acids in length.

In one embodiment, an anti-14-3-3 eta antibody of the invention does not bind to an epitope located at the N-terminus of the human 14-3-3 eta protein.

In a preferred embodiment, an anti-14-3-3 eta antibody binds to a 14-3-3 peptide that is a 14-3-3 eta loop peptide. In a preferred embodiment, the 14-3-3 eta loop peptide comprises an amino acid sequence selected from the group consisting of SEQ ID NOs:11-16. In another embodiment, the 14-3-3 eta loop peptide consists essentially of an amino acid sequence selected from the group consisting of SEQ ID NOs:11-16. In another embodiment, an anti-14-3-3 eta antibody binds to a region of 14-3-3 eta that overlaps with an amino acid sequence corresponding to a sequence selected from the group consisting of SEQ ID NOs:11-16.

In another preferred embodiment, an anti-14-3-3 eta antibody binds to a 14-3-3 peptide that is a 14-3-3 eta helix peptide. In a preferred embodiment, the 14-3-3 eta helix peptide comprises an amino acid sequence selected from the group consisting of SEQ ID NOs:1-10. In another embodiment, the 14-3-3 eta helix peptide consists essentially of an amino acid sequence selected from the group consisting of SEQ ID NOs:1-10. In another embodiment, an anti-14-3-3 eta antibody binds to a region of 14-3-3 eta that overlaps with an amino acid sequence corresponding to a sequence selected from the group consisting of SEQ ID NOs:1-10.

In another preferred embodiment, an anti-14-3-3 eta antibody binds to a 14-3-3 peptide that is a non-helix 14-3-3 eta peptide. In a preferred embodiment, the non-helix 14-3-3 eta peptide comprises an amino acid sequence selected from the group consisting of SEQ ID NOs: 17-32. In another embodiment, the non-helix 14-3-3 eta peptide consists essentially of an amino acid sequence selected from the group consisting of SEQ ID NOs:17-32. In another embodiment, an anti-14-3-3 eta antibody binds to a region of 14-3-3 eta that overlaps with an amino acid sequence corresponding to a sequence selected from the group consisting of SEQ ID NOs:17-32.

In an especially preferred embodiment, an anti-14-3-3 eta antibody of the invention binds to an amino acid sequence selected from the group consisting of LDKFLIKNSNDF(SEQ ID NO:30), KKLEKVKAYR (SEQ ID NO:31), and KNSVVEASEAAYKEA (SEQ ID NO:32).

Exemplary 14-3-3 eta loop, helix, and non-helix peptides are disclosed in Table 1 herein. Notably, SEQ ID NO:30 varies from corresponding 14-3-3 eta sequence in that a cysteine occurring in 14-3-3 eta sequence has been replaced by serine to avoid disulfide bond formation. In one embodiment, the invention provides antibodies that also bind to the natural 14-3-3 sequence correlate of SEQ ID NO:30 comprising a cysteine. In one embodiment, the invention provides antibodies capable of binding to peptide sequences that vary from those listed in the epitope tables herein by substitution of serine for cysteine.

In a preferred embodiment, an anti-14-3-3 antibody is capable of discriminating between 14-3-3 protein isoforms.

In a preferred embodiment, an anti-14-3-3 antibody is a monoclonal antibody.

In a preferred embodiment, an anti-14-3-3 antibody is a humanized antibody.

In one aspect, the invention provides novel 14-3-3 antagonists. In one embodiment, the invention provides novel 14-3-3 antagonist peptides. In another embodiment, the invention provides novel anti-14-3-3 antibodies. In one aspect, the invention provides methods of making 14-3-3 antagonists.

In one aspect, the invention provides nucleic acids encoding 14-3-3 antagonists that are peptides or antibodies. Also provided are vectors, including expression vectors, comprising such nucleic acids. Also provided are host cells comprising such nucleic acids and host cells comprising such vectors. Also provided are methods of making a 14-3-3 antagonist, which comprise the use of such host cells.

In one aspect, the invention provides cells capable of producing 14-3-3 antagonists.

In one embodiment, the cell is a hybridoma, and the 14-3-3 antagonist is an anti-14-3-3 antibody.

In another embodiment, the cell is a genetically modified fibroblast or FLS cell, and the 14-3-3 antagonist is a peptide or an anti-14-3-3 antibody.

In one aspect, the invention provides methods of screening for a 14-3-3 antagonist. In one embodiment, the methods comprise screening candidate agents for the ability to inhibit binding of a 14-3-3 eta protein or 14-3-3 gamma protein to 14-3-3 eta ligand or a 14-3-3 gamma ligand, respectively. In one embodiment, the ligand is a 14-3-3 antagonist peptide. In one embodiment, the ligand is an anti-14-3-3 antibody. In one embodiment, the ligand is an intracellular 14-3-3 binding partner. In one embodiment, the methods comprise analyzing the ability of the candidate agent to inhibit induction of MMP by a 14-3-3 protein.

In one embodiment, a 14-3-3 antagonist competitively inhibits the binding of a 14-3-3 eta protein or a 14-3-3 gamma protein to an anti-14-3-3 antibody or a 14-3-3 antagonist peptide disclosed herein. In one embodiment, the 14-3-3 antagonist is a small molecule chemical composition.

In a preferred embodiment, a 14-3-3 antagonist binds to a region of 14-3-3 protein that is capable of binding to R-18. In a preferred embodiment, a 14-3-3 antagonist binds to a region of 14-3-3 protein that is capable binding to an intracellular 14-3-3 binding partner, preferably Raf. In one embodiment, a 14-3-3 antagonist binds to a 14-3-3 protein without inhibiting the binding of an intracellular 14-3-3 binding partner.

In one aspect, the invention provides pharmaceutical compositions for the treatment of arthritis. The pharmaceutical compositions comprise one or more 14-3-3 antagonists. The pharmaceutical compositions are formulated to provide for engagement of extracellular 14-3-3 protein by the 14-3-3 antagonist.

In one aspect, the invention provides methods for preparing a medicament useful for treating arthritis. Such a medicament comprises one or more 14-3-3 antagonists. The medicament is formulated to provide for engagement of extracellular 14-3-3 protein by the 14-3-3 antagonist.

In one aspect, the invention accordingly involves the use of a 14-3-3 antagonist, such as a 14-3-3 antagonist that is capable of specifically binding to an extracellularly-localized 14-3-3 protein and inhibiting the activity of the 14-3-3 protein, for treating arthritis or to formulate a medicament for treating arthritis.

### BRIEF DESCRIPTION OF THE DRAWINGS

Figure 1. ELISA: Test Bleed Titration of Mouse Anti-AUG1-CLDK Immune Serum (after 2nd boost) on AUG1-CLDK-BSA Antigen (IgG response only).
Figure 2. ELISA: Test Bleed Titration of Mouse Anti- AUG2-KKLE Immune Serum (after 2nd boost) on AUG2-KKLE-BSA antigen (IgG response only).
Figure 3. ELISA: Test Bleed Titration of Mouse Anti-AUG3-CKNS Immune Serum (after 2nd boost) on AUG3-CKNS-BSA Antigen (IgG response only).
Figure 4. Sequence alignment for various 14-3-3 protein isoforms.
Figure 5. R-18 interacts with extracellular 14-3-3 protein and inhibits induction of MMP-1 expression induced by extracellular 14-3-3 protein.
Figure 6. Western Blot showing cell lysate-derived 14-3-3 eta protein and human recombinant 14-3-3 eta immunoprecipated by monoclonal antibody raised against full length human recombinant 14-3-3 eta.
Figure 7. Western Blot showing cell lysate-derived 14-3-3 eta protein and human recombinant 14-3-3 eta immunoprecipated by monoclonal antibody raised against a human 14-3-3 eta peptide fragment 142-158 SEQ ID NO:24 from a non-helical region of the protein.
Figure 8. ELISA: Test Bleed Titration of Mouse anti-14-3-3 eta Immune Sera (after 2nd boost) on 14-3-3 eta Antigen (IgG response only)

### DETAILED DESCRIPTION OF THE INVENTION

The invention provides methods of treating arthritis, including methods of treating ankylosing spondylitis, Behçet's Disease, diffuse idiopathic skeletal hyperostosis (DISH), Ehlers-Danlos Syndrome (EDS), Felty's Syndrome, fibromyalgia, gout, infectious arthritis, juvenile arthritis, lupus, mixed connective tissue disease (MCTD), osteoarthritis, Paget's Disease, polymyalgia rheumatica, polymyositis and dermatomyositis, pseudogout, psoriatic arthritis, Raynaud's Phenomenon, reactive arthritis, rheumatoid arthritis, scleroderma, Sjögren's Syndrome, Still's Disease, and Wegener's granulomatosis.

As used herein, 'arthritis' or 'arthralgia' refer to an inflammatory disorder of the joints of the body. Pain, swelling, stiffness and difficulty of movement are frequently associated with arthritis diseases. Arthritis may result from any of several causes including infection, trauma, degenerative disorders, metabolic disorders or disturbances or other unknown etiologies.

The progression or severity of arthritis in a patient may be measured or quantified using techniques known in the art. As an example, a "Disease Activity Score" (DAS) may be utilized to measure the activity or state of arthritis in a patient. DAS is one of several standards or scores used in clinical practice. A calculation of a DAS may include the following parameters: Number of joints tender to the touch (TEN), number of swollen joints (SW), erythrocyte sedimentation rate (ESR) and patient assessment of disease activity (VAS). Alternatively, a DAS may include C-reactive protein marker assessment (CRP) (Skogh T et al 2003. Ann Rheum Dis 62:681-682). Alternately, one may utilize diagnostic biomarkers, including 14-3-3 eta and/or gamma, to measure the presence or absence of disease, or to determine disease severity.

In the present description the terms "treatment" or "treat" refer to both prophylactic or preventative treatment as well as curative or disease modifying treatment, including treatment of a patient at risk of contracting the disease or suspected to have contracted the disease as well as patients who are ill or have been diagnosed as suffering from a disease or medical condition, and includes suppression of clinical relapse.

The methods involve administration of one or more 14-3-3 antagonists. A 14-3-3 antagonist of the invention binds to a 14-3-3 protein, particularly 14-3-3 eta or gamma, and antagonizes the activity thereof.

As used herein, an 'isoform' refers to two or more functionally similar proteins that have a similar but not identical amino acid sequence and are either encoded by different genes or by different RNA transcripts, primary or processed, from the same gene.

Reference to a 14-3-3 eta protein or a 14-3-3 gamma protein may include fragments thereof. For example, in one embodiment the invention provides methods of screening for a 14-3-3 antagonist which, in a preferred embodiment, comprise screening candidate agents for the ability to inhibit binding of a 14-3-3 ligand to a 14-3-3 protein. It will be understood that an appropriate fragment of a 14-3-3 protein can be used in the assay.

An example of a 14-3-3 antagonist is the R18 inhibitory peptide (Wang et al. 1999 - REF 35). The R18 peptide, also referred to herein as 'R18', is a small peptide which is capable of blocking the association of 14-3-3 proteins with Raf-1. Other examples of peptides that bind to 14-3-3 proteins are known (see, for example, Wang et al. 1999 - REF 35, *infra*; Yaffe et al., Cell, 91:961-971, 1997; Shaw et al., U.S. 5,948,765; Petosa et al., JBC 273:16305-16310, 1998; Fu et al., US 2004/0152630). These and others when formulated to engage aberrantly localized extracellular 14-3-3 protein may find utility in the present invention as therapeutics for the treatment of arthritis.

"Antibody" refers to a composition comprising a protein that binds specifically to a corresponding antigen and has a common, general structure of immunoglobulins. The term antibody specifically covers polyclonal antibodies, monoclonal antibodies, dimers, multimers, multispecific antibodies (e.g., bispecific antibodies), and antibody fragments, so long as they exhibit the desired biological activity. Antibodies may be murine, human, humanized, chimeric, or derived from other species. Typically, an antibody will comprise at least two heavy chains and two light chains interconnected by disulfide bonds, which when combined form a binding domain that interacts with an antigen. Each heavy chain is comprised of a heavy chain variable region (VH) and a heavy chain constant region (CH). The heavy chain constant region is comprised of three domains, CH1, CH2 and CH3, and may be of the mu, delta, gamma, alpha or epsilon isotype. Similarly, the light chain is comprised of a light chain variable region (VL) and a light chain constant region (CL). The light chain constant region is comprised of one domain, CL, which may be of the kappa or lambda isotype. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. The heavy chain constant region mediates binding of the immunoglobulin to host tissue or host factors, particularly through cellular receptors such as the Fc receptors (e.g., FcγRI, FcγRII, FcγRIII, etc.). As used herein, antibody also includes an antigen binding portion of an immunoglobulin that retains the ability to bind antigen. These include, as examples, F(ab), a monovalent fragment of VL CL and VH CH antibody domains; and F(ab')₂ fragment, a bivalent fragment comprising two Fab fragments linked by a disulfide bridge at the hinge region. The term antibody also refers to recombinant single chain Fv fragments (scFv) and bispecific molecules such as, e.g., diabodies, triabodies, and tetrabodies (see, e.g., U.S. Patent No. 5,844,094).

Antibodies may be produced and used in many forms, including antibody complexes. As used herein, the term "antibody complex" refers to a complex of one or more antibodies with another antibody or with an antibody fragment or fragments, or a complex of two or more antibody fragments. Antibody complexes include multimeric forms of anti-14-3-3 antibodies such as homoconjugates and heteroconjugates as well as other cross-linked antibodies as described herein.

"Antigen" is to be construed broadly and refers to any molecule, composition, or particle that can bind specifically to an antibody. An antigen has one or more epitopes that interact with the antibody, although it does not necessarily induce production of that antibody.

The terms "cross-linked", "cross-linking" and grammatical equivalents thereof, refer to the attachment of two or more antibodies to form antibody complexes, and may also be referred to as multimerization. Cross-linking or multimerization includes the attachment of two or more of the same antibodies (e.g. homodimerization), as well as the attachment of two or more different antibodies (e.g. heterodimerization). Those of skill in the art will also recognize that cross-linking or multimerization is also referred to as forming antibody homoconjugates and antibody heteroconjugates. Such conjugates may involve the attachment of two or more monoclonal antibodies of the same clonal origin (homoconjugates) or the attachment of two or more antibodies of different clonal origin (also referred to as heteroconjugates or bispecific). Antibodies may be crosslinked by non-covalent or covalent attachment. Numerous techniques suitable for cross-linking will be appreciated by those of skill in the art. Non-covalent attachment may be achieved through the use of a secondary antibody that is specific to the primary antibody species. For example, a goat anti-mouse (GAM) secondary antibody may be used to cross-link a mouse monoclonal antibody. Covalent attachment may be achieved through the use of chemical cross-linkers.

"Epitope" refers to a determinant capable of specific binding to an antibody. Epitopes are chemical features generally present on surfaces of molecules and accessible to interaction with an antibody. Typical chemical features are amino acids and sugar moieties, having three-dimensional structural characteristics as well as chemical properties including charge, hydrophilicity, and lipophilicity. Conformational epitopes are distinguished from non-conformational epitopes by loss of reactivity with an antibody following a change in the spatial elements of the molecule without any change in the underlying chemical structure.

"Humanized antibody" refers to an immunoglobulin molecule containing a minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. A humanized antibody will also encompass immunoglobulins comprising at least a portion of an immunoglobulin constant region (Fc), generally that of a human immunoglobulin (Jones et al., Nature 321:522-525 (1986); Reichmann et al, Nature 332:323-329 (1988)).

"Immunogen" refers to a substance, compound, or composition which stimulates the production of an immune response.

The term "immunoglobulin locus" refers to a genetic element or set of linked genetic elements that comprise information that can be used by a B cell or B cell precursor to express an immunoglobulin polypeptide. This polypeptide can be a heavy chain polypeptide, a light chain polypeptide, or the fusion of a heavy and a light chain polypeptide. In the case of an unrearranged locus, the genetic elements are assembled by a B cell precursor to form the gene encoding an immunoglobulin polypeptide. In the case of a rearranged locus, a gene encoding an immunoglobulin polypeptide is contained within the locus.

"Isotype" refers to an antibody class defined by its heavy chain constant region. Heavy chains are generally classified as gamma, mu, alpha, delta, epsilon and designated as IgG, IgM, IgA, IgD, and IgE. Variations within each isotype are categorized into subtypes, for example subtypes of IgG are divided into IgG1, IgG2, IgG3, and IgG4, while IgA is divided into IgA1 and IgA2. The IgY isotype is specific to birds.

"Monoclonal antibody" or "monoclonal antibody composition" refers to a preparation of antibody molecules of single molecular composition. A monoclonal antibody composition displays a single binding specificity and affinity for a particular epitope.

The term "human monoclonal antibody" includes antibodies displaying a single binding specificity which have variable and/or constant regions (if present) derived from human immunoglobulin sequences. In one embodiment, the human monoclonal antibodies are produced by a hybridoma which includes a B cell obtained from a transgenic non-human animal, e.g., a transgenic mouse, having a genome comprising a human heavy chain transgene and a light chain transgene, fused to an immortalized cell.

"Single chain Fv" or "scFv" refers to an antibody comprising the VH and VL regions of an antibody, wherein these domains are present in a single polypeptide chain. Generally, an scFv further comprises a polypeptide linker between the VH and VL domains which enables the scFv to form the desired structure for antigen binding.

"Subject" or "patient" are used interchangeably and refer to, except where indicated, mammals such as humans and non-human primates, as well as rabbits, rats, mice, goats, pigs, and other mammalian species.

"Recombinant antibody" refers to all antibodies produced by recombinant techniques. These include antibodies obtained from an animal that is transgenic for the immunoglobulin locus, antibodies expressed from a recombinant expression vector, or antibodies created, prepared, and expressed by splicing of any immunoglobulin gene sequence to any other nucleic acid sequence.

### Anti-14-3-3 Antibodies

In one aspect, the invention provides novel anti-14-3-3 antibodies that bind specifically to 14-3-3 eta or 14-3-3 gamma protein. Preferably, an anti-14-3-3 antibody of the invention is capable of specifically binding to 14-3-3 protein in its natural 3-D configuration. By specifically binding to a 14-3-3 protein in its "natural configuration" is meant an ability to bind to 14-3-3 protein as encountered in vivo. This may be evidenced, for example, by the ability of antibody to immunoprecipitate 14-3-3 eta protein from a biological sample.

In a preferred embodiment, an anti-14-3-3 eta antibody of the invention is capable of binding to 14-3-3 protein that is aberrantly localized in the extracellular synovial space in arthritis. This may be evidenced, for example, by immunoprecipitation of 14-3-3 protein present in a synovial fluid sample from a patient having arthritis.

In a preferred embodiment, an anti-14-3-3 antibody of the invention is capable of discriminating between 14-3-3 protein isoforms. Such antibodies have an ability to bind specifically to a particular 14-3-3 protein isoform and bind preferentially to that isoform over other 14-3-3 protein isoforms under the same conditions. This may be evidenced, for example, using an ELISA assay, which may be done using, for example, supernatant from hybridoma clones. A control (e.g., pre-immune serum) is preferably used. A "selective" antibody is capable of recognizing a particular 14-3-3 isoform and generating a higher signal against that isoform as compared to other isoforms, preferably at least a 1.5 fold, more preferably at least a 2 fold higher signal as compared to other isoforms. In a preferred embodiment, a selective antibody has an ability to selectively immunoprecipitate the particular 14-3-3 eta as compared to other 14-3-3 isoforms.

In a preferred embodiment, the anti-14-3-3 antibody exhibits such selectivity for 14-3-3 eta protein over 14-3-3 alpha, beta, delta, epsilon, gamma, tau, and zeta proteins. This may be evidenced, for example, by ELISA.

In another preferred embodiment, the anti-14-3-3 antibody exhibits such selectivity for 14-3-3 gamma protein over 14-3-3 alpha, beta, delta, epsilon, eta, tau, and zeta proteins. This may be evidenced, for example, by ELISA.

In a preferred embodiment, an anti-14-3-3 antibody of the invention is a 14-3-3 antagonist, though other anti-14-3-3 antibodies are also contemplated within the scope of the invention.

In a preferred embodiment, an anti-14-3-3 antibody is capable of inhibiting the induction of MMP by 14-3-3 protein, particularly 14-3-3 gamma or 14-3-3 eta. Preferably, the MMP is selected from the group consisting of MMP-1, 3, 8, 9, 10, 11 and 13, with MMP-1 and MMP-3 being especially preferred. Such capability may be determined by an in vitro assay or in vivo assay. As will be appreciated by one of skill in the art, the assays will be designed such that in the absence of anti-14-3-3 antibody, the presence of 14-3-3 protein will result in the induction of MMP. An ability to reduce this induction of MMP by 14-3-3 protein can evidence such a function-inhibiting capability for an anti-14-3-3 antibody.

In one aspect, the invention provides anti-14-3-3 eta antibodies. In a preferred embodiment, the anti-14-3-3 eta antibody binds to a 14-3-3 peptide comprising a segment of the amino acid sequence set forth in SEQ ID NO:63. In a preferred embodiment, the segment is at least 6, more preferably at least 7, more preferably at least 8 amino acids in length.

In one embodiment, an anti-14-3-3 eta antibody of the invention does not bind to an epitope located at the N-terminus of the human 14-3-3 eta protein.

In a preferred embodiment, an anti-14-3-3 eta antibody binds to a 14-3-3 peptide that is a 14-3-3 eta loop peptide. In a preferred embodiment, the 14-3-3 eta loop peptide comprises an amino acid sequence selected from the group consisting of SEQ ID NOs:11-16. In another embodiment, the 14-3-3 eta loop peptide consists essentially of an amino acid sequence selected from the group consisting of SEQ ID NOs:11-16. In another embodiment, an anti-14-3-3 eta antibody binds to a region of 14-3-3 eta that overlaps with an amino acid sequence corresponding to a sequence selected from the group consisting of SEQ ID NOs:11-16.

In another preferred embodiment, an anti-14-3-3 eta antibody binds to a 14-3-3 peptide that is a 14-3-3 eta helix peptide. In a preferred embodiment, the 14-3-3 eta helix peptide comprises an amino acid sequence selected from the group consisting of SEQ ID NOs:1-10. In another embodiment, the 14-3-3 eta helix peptide consists essentially of an amino acid sequence selected from the group consisting of SEQ ID NOs:1-10. In another embodiment, an anti-14-3-3 eta antibody binds to a region of 14-3-3 eta that overlaps with an amino acid sequence corresponding to a sequence selected from the group consisting of SEQ ID NOs:1-10.

In another preferred embodiment, an anti-14-3-3 eta antibody binds to a 14-3-3 peptide that is a non-helix 14-3-3 eta peptide. In a preferred embodiment, the non-helix 14-3-3 eta peptide comprises an amino acid sequence selected from the group consisting of SEQ ID NOs: 17-32. In another embodiment, the non-helix 14-3-3 eta peptide consists essentially of an amino acid sequence selected from the group consisting of SEQ ID NOs:17-32. In another embodiment, an anti-14-3-3 eta antibody binds to a region of 14-3-3 eta that overlaps with an amino acid sequence corresponding to a sequence selected from the group consisting of SEQ ID NOs:17-32.

In an especially preferred embodiment, an anti-14-3-3 eta antibody of the invention binds to an amino acid sequence selected from the group consisting of LDKFLIKNSNDF(SEQ ID NO:30), KKLEKVKAYR (SEQ ID NO:31), and KNSVVEASEAAYKEA (SEQ ID NO:32).

Exemplary 14-3-3 eta loop, helix, and non-helix peptides are disclosed in Table 1 herein. Notably, SEQ ID NO:30 varies from corresponding 14-3-3 eta sequence in that a cysteine occurring in 14-3-3 eta sequence has been replaced by serine to avoid disulfide bond formation. In one embodiment, the invention provides antibodies that also bind to the natural 14-3-3 sequence correlate of SEQ ID NO:30 comprising a cysteine. In one embodiment, the invention provides antibodies capable of binding to peptide sequences that vary from those listed in the epitope tables herein by substitution of serine for cysteine.

In one aspect, the invention provides anti-14-3-3 gamma antibodies. In a preferred embodiment, the anti-14-3-3 gamma antibody binds to a 14-3-3 peptide comprising a segment of the amino acid sequence set forth in SEQ ID NO:64. In a preferred embodiment, the segment is at least 6, more preferably at least 7, more preferably at least 8 amino acids in length.

In a preferred embodiment, an anti-14-3-3 gamma antibody binds to a 14-3-3 peptide that is a 14-3-3 gamma loop peptide. In a preferred embodiment, the 14-3-3 gamma loop peptide comprises an amino acid sequence selected from the group consisting of SEQ ID NOs:44-49. In another embodiment, the 14-3-3 gamma loop peptide consists essentially of an amino acid sequence selected from the group consisting of SEQ ID NOs:44-49. In another embodiment, an anti-14-3-3 gamma antibody binds to a region of 14-3-3 gamma that overlaps with an amino acid sequence corresponding to a sequence selected from the group consisting of SEQ ID NOs:44-49.

In another preferred embodiment, an anti-14-3-3 gamma antibody binds to a 14-3-3 peptide that is a 14-3-3 gamma helix peptide. In a preferred embodiment, the 14-3-3 gamma helix peptide comprises an amino acid sequence selected from the group consisting of SEQ ID NOs:33-43. In another embodiment, the 14-3-3 gamma helix peptide consists essentially of an amino acid sequence selected from the group consisting of SEQ ID NOs:33-43. In another embodiment, an anti-14-3-3 gamma antibody binds to a region of 14-3-3 gamma that overlaps with an amino acid sequence corresponding to a sequence selected from the group consisting of SEQ ID NOs:33-43.

In another preferred embodiment, an anti-14-3-3 gamma antibody binds to a 14-3-3 peptide that is a non-helix 14-3-3 gamma peptide. In a preferred embodiment, the non-helix 14-3-3 gamma peptide comprises an amino acid sequence selected from the group consisting of SEQ ID NOs:50-62. In another embodiment, the non-helix 14-3-3 gamma peptide consists essentially of an amino acid sequence selected from the group consisting of SEQ ID NOs:50-62. In another embodiment, an anti-14-3-3 gamma antibody binds to a region of 14-3-3 gamma that overlaps with an amino acid sequence corresponding to a sequence selected from the group consisting of SEQ ID NOs:50-62.

### Monoclonal Antibodies, Hybridomas, and Methods of Making the Same

The present disclosure provides monoclonal antibodies that specifically bind to 14-3-3 eta protein, as well as monoclonal antibodies that specifically bind to 14-3-3 gamma protein. Also provided are hybridoma cell lines capable of producing such antibodies.

In one aspect, the invention provides monoclonal anti-14-3-3 eta antibodies that bind to a 14-3-3 eta loop, helix, or non-helix peptide. In a preferred embodiment, the peptide comprises an amino acid sequence selected from the group consisting of SEQ ID NOs:1-32. In an especially preferred embodiment, the invention provides anti-14-3-3 eta monoclonal antibodies that specifically bind to an amino acid sequence selected from the group consisting of LDKFLIKNSNDF(SEQ ID NO:30), KKLEKVKAYR (SEQ ID NO:31), and KNSVVEASEAAYKEA (SEQ ID NO:32). Also provided are hybridoma cell lines capable of producing such antibodies.

In one aspect, the invention provides hybridomas produced by fusion of a spleen cell derived from a mouse immunized with an immunogen comprising a 14-3-3 eta loop, helix, or non-helix peptide. In a preferred embodiment, the peptide comprises an amino acid sequence selected from the group consisting of SEQ ID NOs:1-32. In an especially preferred embodiment, the invention provides hybridomas produced by fusion of spleen cells derived from mice immunized with an immunogen comprising LDKFLIKNSNDF(SEQ ID NO:30), KKLEKVKAYR (SEQ ID NO:31), or KNSVVEASEAAYKEA (SEQ ID NO:32). Also provided are monoclonal antibodies produced by such hybridomas.

In a preferred embodiment, the invention provides monoclonal anti-14-3-3 gamma antibodies that bind to a 14-3-3 gamma loop, helix, or non-helix peptide. In a preferred embodiment, the peptide comprises an amino acid sequence selected from the group consisting of SEQ ID NOs:33-62. Also provided are hybridoma cell lines capable of producing such antibodies.

In one aspect, the invention provides hybridomas produced by fusion of a spleen cell derived from a mouse immunized with an immunogen comprising a 14-3-3 gamma loop, helix, or non-helix peptide. In a preferred embodiment, the peptide comprises an amino acid sequence selected from the group consisting of SEQ ID NOs:33-62. Also provided are monoclonal antibodies produced by such hybridomas.

The present disclosure further provides methods of producing such monoclonal antibodies, or derivatives thereof, comprising cultivating a hybridoma of the invention under suitable conditions, whereby a monoclonal antibody is produced, and obtaining the antibody and/or derivative thereof from the cell and/or from the cell culture medium.

Antibodies can be produced readily by one skilled in the art. The general methodology for making monoclonal antibodies by hybridomas is now well known to the art. See, e.g., M. Schreier et al., Hybridoma Techniques (Cold Spring Harbor Laboratory) 1980; Hammerling et al., Monoclonal Antibodies and T-Cell Hybridomas (Elsevier Biomedical Press) 1981.

In some embodiments, these methods comprise cultivating a hybridoma cell under suitable conditions wherein the antibody is produced, and obtaining the antibody and/or derivative thereof from the cell and/or from the cell culture medium.

The present invention also contemplates the use of phage libraries to pan for antibodies capable of binding to the 14-3-3 peptides of interest described herein. For example, see Konthur et al., Targets, 1: 30-36, 2002.

The antibodies produced by any means can be purified by methods known to the skilled artisan. Purification methods include, among others, selective precipitation, liquid chromatography, HPLC, electrophoresis, chromatofocusing, and various affinity techniques. Selective precipitation may use ammonium sulfate, ethanol (Cohn precipitation), polyethylene glycol, or other agents available in the art. Liquid chromatography mediums, include, among others, ion exchange medium DEAE, polyaspartate, hydroxylapatite, size exclusion (e.g., those based on crosslinked agarose, acrylamide, dextran, etc.), hydrophobic matrices (e.g., Blue Sepharose). Affinity techniques typically rely on proteins that interact with the immunoglobulin Fc domain. Protein A from Staphylococcus aureas can be used to purify antibodies that are based on human γ1, γ2, or γ4 heavy chains (Lindmark et al., J. Immunol. Meth. 62:1-13 (1983)). Protein G from C and G streptococci is useful for all mouse isotypes and for human γ3 (Guss et al., EMBO J. 5:15671575 (1986)). Protein L, a Peptostreptococcus magnus cell-wall protein that binds immunoglobulins (Ig) through k light-chain interactions (BD Bioscience/ClonTech. Palo Alto, CA), is useful for affinity purification of Ig subclasses IgM, IgA, IgD, IgG, IgE and IgY. Recombinant forms of these proteins are also commercially available. If the antibody contains metal binding residues, such as phage display antibodies constructed to contain histidine tags, metal affinity chromatography may be used. When sufficient amounts of specific cell populations are available, antigen affinity matrices may be made with the cells to provide an affinity method for purifying the antibodies.

In a preferred embodiment, isolation involves affinity chromatography using an appropriate 14-3-3 protein or fragment thereof.

The present invention provides the antibodies described herein, as well as corresponding antibody fragments and antigen-binding portions. All are encompassed by the term anti-14-3-3 antibody. The terms "antibody fragment" or "antigen-binding portion" of an antibody (or simply "antibody portion") of the present invention, as used herein, refers to one or more fragments of an antibody that retain the ability to specifically bind to an antigen. It has been shown that the antigen-binding function of an antibody can be performed by fragments of a full-length antibody. Examples of binding fragments encompassed within the term "antibody fragment" or "antigen-binding portion" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (e.g., Ward et al., (1989) Nature 341:544-546), which consists of a VH domain; and (vi) an isolated complementarity determining region (CDR), and (vii) bispecific single chain Fv dimers (e.g., PCT/US92/09965). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. The antibody fragments may be modified. For example, the molecules may be stabilized by the incorporation of disulphide bridges linking the VH and VL domains (Reiter et al., 1996, Nature Biotech. 14:1239-1245).

Immunoglobulin molecules can be cleaved into fragments. The antigen binding region of the molecule can be divided into either F(ab')2 or Fab fragments. The F(ab')2 fragment is divalent and is useful when the Fc region is either undesirable or not a required feature. The Fab fragment is univalent and is useful when an antibody has a very high avidity for its antigen. Eliminating the Fc region from the antibody decreases non-specific binding between the Fc region and Fc receptor bearing cells. To generate Fab or F(ab')2 fragments, the antibodies are digested with an enzyme. Proteases that cleave at the hinge region of an immunoglobulin molecule preserve the disulfide bond(s) linking the Fab domains such that they remain together following cleavage. A suitable protease for this purpose is pepsin. For producing Fab fragments, proteases are chosen such that cleavage occurs above the hinge region containing the disulfide bonds that join the heavy chains but which leaves intact the disulfide bond linking the heavy and light chain. A suitable protease for making Fab fragments is papain. The fragments are purified by the methods described above, with the exception of affinity techniques requiring the intact Fc region (e.g., Protein A affinity chromatography).

Antibody fragments can be produced by limited proteolysis of antibodies and are called proteolytic antibody fragments. These include, but are not limited to, the following: F(ab')2 fragments, Fab' fragments, Fab'-SH fragments, and Fab fragments. "F(ab')2 fragments" are released from an antibody by limited exposure of the antibody to a proteolytic enzyme, e.g., pepsin or ficin. An F(ab')2 fragment comprises two "arms," each of which comprises a variable region that is directed to and specifically binds a common antigen. The two Fab' molecules are joined by interchain disulfide bonds in the hinge regions of the heavy chains; the Fab' molecules may be directed toward the same (bivalent) or different (bispecific) epitopes. "Fab' fragments" contain a single antigen-binding domain comprising an Fab and an additional portion of the heavy chain through the hinge region. "Fab'-SH fragments" are typically produced from F(ab')2 fragments, which are held together by disulfide bond(s) between the H chains in an F(ab')2 fragment. Treatment with a mild reducing agent such as, by way of non-limiting example, beta-mercaptoethylamine, breaks the disulfide bond(s), and two Fab' fragments are released from one F(ab')2 fragment. Fab'-SH fragments are monovalent and monospecific. "Fab fragments" (i.e., an antibody fragment that contains the antigen-binding domain and comprises a light chain and part of a heavy chain bridged by a disulfide bond) may be produced by papain digestion of intact antibodies. A convenient method is to use papain immobilized on a resin so that the enzyme can be easily removed and the digestion terminated. Fab fragments do not have the disulfide bond(s) between the H chains present in an F(ab')2 fragment.

"Single-chain antibodies" are one type of antibody fragment. The term single chain antibody is often abbreviated as "scFv" or "sFv." These antibody fragments are produced using recombinant DNA technology. A single-chain antibody consists of a polypeptide chain that comprises both a V_{H} and a V_{L} domains which interact to form an antigen-binding site. The V_{H} and V_{L} domains are usually linked by a peptide of 10 to 25 amino acid residues.

The term "single-chain antibody" further includes but is not limited to a disulfide-linked Fv (dsFv) in which two single-chain antibodies (each of which may be directed to a different epitope) are linked together by a disulfide bond; a bispecific sFv in which two discrete scFvs of different specificity are connected with a peptide linker; a diabody (a dimerized sFv formed when the V_{H} domain of a first sFv assembles with the V_{L} domain of a second sFv and the V_{L} domain of the first sFv assembles with the V_{H} domain of the second sFv; the two antigen-binding regions of the diabody may be directed towards the same or different epitopes); and a triabody (a trimerized sFv, formed in a manner similar to a diabody, but in which three antigen-binding domains are created in a single complex; the three antigen binding domains may be directed towards the same or different epitopes).

"Complementary determining region peptides" or "CDR peptides" are another form of an antibody fragment. In one embodiment, the invention provides such CDR peptides that are 14-3-3 antagonists. A CDR peptide (also known as "minimal recognition unit") is a peptide corresponding to a single complementarity-determining region (CDR), and can be prepared by constructing genes encoding the CDR of an antibody of interest. Such genes are prepared, for example, by using the polymerase chain reaction to synthesize the variable region from RNA of antibody-producing cells. See, for example, Larrick et al., Methods: A Companion to Methods in Enzymology 2:106, 1991.

In "cysteine-modified antibodies," a cysteine amino acid is inserted or substituted on the surface of antibody by genetic manipulation and used to conjugate the antibody to another molecule via, e.g., a disulfide bridge. Cysteine substitutions or insertions for antibodies have been described (see U.S. Pat. No. 5,219,996). Methods for introducing Cys residues into the constant region of the IgG antibodies for use in site-specific conjugation of antibodies are described by Stimmel et al. (J. Biol. Chem 275:330445-30450, 2000).

The present disclosure further provides humanized and non-humanized antibodies. Humanized forms of non-human (e.g., mouse) antibodies are chimeric antibodies that contain minimal sequence derived from non-human immunoglobulin. Generally, humanized antibodies are non-human antibodies that have had the variable-domain framework regions swapped for sequences found in human antibodies. The humanized antibodies may be human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin.

Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536. Humanized antibodies can also be generated using mice with a genetically engineered immune system. e.g., Roque et al., 2004, Biotechnol. Prog. 20:639-654.

It can be desirable to modify the antibodies of the invention with respect to effector function. For example, cysteine residue(s) can be introduced into the Fc region, thereby allowing interchain disulfide bond formation in this region. Homodimeric antibodies can also be prepared using heterobifunctional cross-linkers, e.g., Wolff et al. Cancer Research, 53:2560-2565 (1993). Alternatively, an antibody can be engineered that has dual Fc regions. See for example Stevenson et al., Anti-Cancer Drug Design, 3:219-230 (1989).

### Modified Antibodies

In one aspect, the present invention provides 14-3-3 antibodies that are modified antibodies which are derived from an antibody that specifically binds a 14-3-3 protein. Modified antibodies also include recombinant antibodies as described herein.

Numerous types of modified or recombinant antibodies will be appreciated by those of skill in the art. Suitable types of modified or recombinant antibodies include, without limitation, engineered monoclonal antibodies (e.g. chimeric monoclonal antibodies, humanized monoclonal antibodies), domain antibodies (e.g. Fab, Fv, VH, scFV, and dsFv fragments), multivalent or multispecific antibodies (e.g. diabodies, minibodies, miniantibodies, (scFV)2, tribodies, and tetrabodies), and antibody conjugates as described herein.

In one aspect, the present invention provides anti-14-3-3 antibodies which are domain antibodies. "Domain antibodies" are functional binding domains of antibodies, corresponding to the variable regions of either the heavy (VH) or light (VL) chains of human antibodies. Domain antibodies may have a molecular weight of approximately 13 kDa, or less than one-tenth the size of a full antibody. They are well expressed in a variety of hosts including bacterial, yeast, and mammalian cell systems. In addition, domain antibodies are highly stable and retain activity even after being subjected to harsh conditions, such as freeze-drying or heat denaturation. See, for example, US Patent 6,291,158; 6,582,915; 6,593,081; 6,172,197; US Serial No. 2004/0110941; European Patent 0368684; US Patent 6,696,245, WO04/058821, WO04/003019 and WO03/002609. In one embodiment, the domain antibody of the present invention is a single domain. Single domain antibodies may be prepared, for example, as described in U.S. Patent No. 6,248,516.

In another aspect, the present invention includes multi-specific antibodies. Multi-specific antibodies include bispecific, trispecific, etc. antibodies. Bispecific antibodies can be produced via recombinant means, for example by using leucine zipper moieties (i.e., from the Fos and Jun proteins, which preferentially form heterodimers; e.g., Kostelny et al., 1992, J. Immnol. 148:1547) or other lock and key interactive domain structures, for example as described in U.S. Pat. No. 5,582,996. Additional useful techniques include those described in U.S. Pat. No. 5,959,083; and U.S. Pat. No. 5,807,706.

Bispecific antibodies are also sometimes referred to as "diabodies." These are antibodies that bind to two (or more) different antigens. Also known in the art are triabodies (a trimerized sFv, formed in a manner similar to a diabody, but in which three antigen-binding domains are created in a single complex; the three antigen binding domains may be directed towards the same or different epitopes) or tetrabodies (four antigen-binding domains created in a single complex where the four antigen binding domains may be directed towards the same or different epitopes). Dia-, tria- and tetrabodies can be manufactured in a variety of ways known in the art (e.g., Holliger and Winter, 1993, Current Opinion Biotechnol. 4:446-449), e.g., prepared chemically or from hybrid hybridomas. In addition, such antibodies and fragments thereof may be constructed by gene fusion (e.g., Tomlinson et. al., 2000, Methods Enzymol. 326:461-479; WO94/13804; Holliger et al., 1993, Proc. Natl. Acad. Sci. U.S.A. 90:6444-6448).

In another embodiment, the present invention provides minibodies, which are minimized antibody-like proteins that include a scFV joined to a CH3 domain, that are derived from an antibody that specifically binds 14-3-3 protein. Minibodies can be made as described in the art (e.g., Hu et al., 1996, Cancer Res. 56:3055-3061).

In another embodiment, the present invention provides 14-3-3 binding domain-immunglobulin fusion proteins. In one embodiment, the fusion protein may include a 14-3-3 binding domain polypeptide fused to an immunoglobulin hinge region polypeptide, which is fused to an immunoglobulin heavy chain CH2 constant region polypeptide fused to an immunoglobulin heavy chain CH3 constant region polypeptide. Under the present invention, 14-3-3 antibody fusion proteins can be made by methods appreciated by those of skill in the art (See for example published U.S. Patent Application Nos. 20050238646, 20050202534, 20050202028, 2005020023, 2005020212, 200501866216, 20050180970, and 20050175614).

In another embodiment, the present invention provides a heavy-chain protein derived from a 14-3-3 antibody. Naturally-occurring heavy chain antibodies (e.g. camelidae antibodies having no light chains) have been utilized to develop antibody-derived therapeutic proteins that typically retain the structure and functional properties of naturally-occurring heavy-chain antibodies. They are known in the art as Nanobodies. Heavy chain proteins derived from a 14-3-3 heavy chain antibody may be made by methods appreciated by those of skill in the art (See for example published U.S. Patent Application Nos. 20060246477, 20060211088, 20060149041, 20060115470, and 20050214857). Further, regarding the production of heavy chain-only antibodies in light chain-deficient mice, see for example Zou et al., JEM, 204:3271-3283,2007.

In one aspect, the present invention provides a modified antibody that is a human antibody. In one embodiment, fully human 14-3-3 antibodies are provided. "Fully human antibody" or "complete human antibody" refers to a human antibody having only the gene sequence of an antibody derived from a human chromosome. The anti-14-3-3 complete human antibody can be obtained by a method using a human antibody-producing mouse having a human chromosome fragment containing the genes for a heavy chain and light chain of a human antibody [see for example Tomizuka, K. et al., Nature Genetics, 16, p.133-143, 1997; Kuroiwa, Y. et al., Nuc. Acids Res., 26, p.3447-3448, 1998; Yoshida, H. et al., Animal Cell Technology: Basic and Applied Aspects vol. 10, p.69-73 (Kitagawa, Y., Matuda, T. and lijima, S. eds.), Kluwer Academic Publishers, 1999; Tomizuka, K. et al., Proc. Natl. Acad. Sci. USA, 97, 722-727, 2000] or obtained by a method for obtaining a human antibody derived from a phage display selected from a human antibody library (see for example Wormstone, I. M. et al., Investigative Ophthalmology & Visual Science. 43(7), p.2301-8, 2002; Carmen, S. et al., Briefings in Functional Genomics and Proteomics, 1 (2), p.189-203, 2002; Siriwardena, D. et al., Ophthalmology, 109(3), p.427-431, 2002).

In one aspect, the present invention provides a 14-3-3 antibody that is an antibody analog, sometimes referred to as "synthetic antibodies." For example, alternative protein scaffolds or artificial scaffolds with grafted CDRs may be used. Such scaffolds include, but are not limited to, synthetic scaffolds consisting, for example, of biocompatible polymers. See, for example, Korndorfer et al., 2003, Proteins: Structure, Function, and Bioinformatics, Volume 53, Issue 1:121-129. Roque et al., 2004, Biotechnol. Prog. 20:639-654. In addition, peptide antibody mimetics ("PAMs") can be used, as well as antibody mimetics utilizing fibronectin components as a scaffold.

In one aspect, the present invention provides cross-linked antibodies that include two or more antibodies described herein attached to each other to form antibody complexes. Cross-linked antibodies are also referred to as antibody multimers, homoconjugates, and heteroconjugates.

In some embodiments, the antibody complexes provided herein include multimeric forms of anti-14-3-3 antibodies. For example, antibody complexes of the invention may take the form of antibody dimers, trimers, or higher-order multimers of monomeric immunoglobulin molecules. Crosslinking of antibodies can be done through various methods know in the art. For example, crosslinking of antibodies may be accomplished through natural aggregation of antibodies, through chemical or recombinant linking techniques or other methods known in the art. For example, purified antibody preparations can spontaneously form protein aggregates containing antibody homodimers, and other higher-order antibody multimers.

In one embodiment, the present invention provides homodimerized antibodies that specifically bind to 14-3-3 antigen.

Antibodies can be cross-linked or dimerized through linkage techniques known in the art. Non-covalent methods of attachment may be utilized. In a specific embodiment, crosslinking of antibodies can be achieved through the use of a secondary crosslinker antibody. The crosslinker antibody can be derived from a different animal compared to the antibody of interest. For example, a goat anti-mouse antibody (Fab specific) may be added to a mouse monoclonal antibody to form a heterodimer. This bivalent crosslinker antibody recognizes the Fab or Fc region of the two antibodies of interest forming a homodimer.

In one embodiment of the present invention, an antibody that specifically binds to 14-3-3 antigen is cross-linked using a goat anti-mouse antibody (GAM). In another embodiment, the GAM crosslinker recognizes the Fab or Fc region of two antibodies, each of which specifically binds a 14-3-3 antigen.

Methods for covalent or chemical attachment of antibodies may also be utilized. Chemical crosslinkers can be homo or heterobifunctional and will covalently bind with two antibodies forming a homodimer. Cross-linking agents are well known in the art; for example, homo-or hetero-bifunctional linkers as are well known (see the 2006 Pierce Chemical Company Crosslinking Reagents Technical Handbook; Hermanson, G.T., Bioconjugate Techniques, Academic Press, San Diego, CA (1996); Aslam M. and Dent AH., Bioconjugation: protein coupling techniques for the biomedical sciences, Houndsmills, England: Macmillan Publishers (1999); Pierce: Applications Handbook & Catalog, Perbio Science, Ermbodegem, Belgium (2003-2004); Haughland, R.P., Handbook of Fluorescent Probes and Research Chemicals Eugene, 9th Ed., Molecular Probes, OR (2003); and U.S. Patent No. 5,747,641) Those of skill in the art will appreciate the suitability of various functional groups on the amino acid(s) of an antibody for modification, including cross-linking. Suitable examples of chemical crosslinkers used for antibody crosslinking include, but not limited to, SMCC [succinimidyl 4-(maleimidomethyl)cyclohexane-1-carboxylate], SATA [N-succinimidyl S-acethylthio-acetate], hemi-succinate esters of N-hydroxysuccinimide; sulfo-N- hydroxy-succinimide; hydroxybenzotriazole, and p-nitrophenol; dicyclohexylcarbodiimide (DCC), 1-(3-dimethylaminopropyl)-3-ethylcarbodiimide (ECD), and 1-(3-dimethylaminopropyl)-3-ethylcarbodiimide methiodide (EDCI) (see, e.g., U.S. Patent No. 4,526,714, the disclosure of which is fully incorporated by reference herein). Other linking reagents include glutathione, 3-(diethoxyphosphoryloxy)-1,2,3-benzotriazin-4(3H)-one (DEPBT), onium salt-based coupling reagents, polyoxyethylene-based heterobifunctional cross-linking reagents, and other reagents (Haitao, et al., Organ Lett 1:91-94 (1999); Albericio et al., J Organic Chemistry 63:9678-9683 (1998); Arpicco et al., Bioconjugate Chem. 8:327-337 (1997); Frisch et al., Bioconjugate Chem. 7:180-186 (1996); Deguchi et al., Bioconjugate Chem. 10:32-37 (1998); Beyer et al., J. Med. Chem. 41:2701-2708 (1998); Drouillat et al., J. Pharm. Sci. 87:25-30 (1998); Trimble et al., Bioconjugate Chem. 8:416-423 (1997)). An exemplary protocols for the formation of antibody homodimers is given in U.S. Patent Publication 20060062786. Techniques for conjugating therapeutic compounds to antibodies are also described in Arnon et al., "Monoclonal Antibodies for Immunotargeting of Drugs in Cancers Therapy," in Monoclonal Antibodies and Cancer Therapy, Reisfeld et al., ed., pp243-256, Alan R. Liss, Inc. (1985); Thorpe, et al. "The Preparation and Cytotoxic Properties of Antibody Toxin Conjugates," Immunol. Rev. 62:119-58 (1982); and Pietersz, G.A., "The linkage of cytotoxic drugs to monoclonal antibodies for the treatment of cancer," Bioconjugate Chemistry 1(2):89-95 (1990), all references incorporated herein by reference.

In addition, the antibody-antibody conjugates of this invention can be covalently bound to each other by techniques known in the art such as the use of the heterobifunctional cross-linking reagents, GMBS (maleimidobutryloxy succinimide), and SPDP (N-succinimidyl 3-(2-pyridyldithio)propionate) [see, e.g., Hardy, "Purification And Coupling Of Fluorescent Proteins For Use In Flow Cytometry", Handbook Of Experimental Immunology, Volume 1, Immunochemistry, Weir et al. (eds.), pp. 31.4-31.12 4th Ed., (1986), and Ledbetter et al. U.S. Patent No. 6,010,902].

In addition, antibodies may be linked via a thioether cross-link as described in U.S. Patent Publication 20060216284, U.S. Patent No. 6,368,596. As will be appreciated by those skilled in the art, antibodies can be crosslinked at the Fab region. In some embodiments, it is desirable that the chemical crosslinker not interact with the antigen-binding region of the antibody as this may affect antibody function.

### Conjugated Antibodies

The 14-3-3 antagonists disclosed herein include antibodies conjugated to inorganic or organic compounds, including, by way of example and not limitation, other proteins, nucleic acids, carbohydrates, steroids, and lipids (see for example Green, et al., Cancer Treatment Reviews, 26:269-286 (2000). The compound may be bioactive. Bioactive refers to a compound having a physiological effect on the cell as compared to a cell not exposed to the compound. A physiological effect is a change in a biological process, including, by way of example and not limitation, DNA replication and repair, recombination, transcription, translation, secretion, membrane turnover, cell adhesion, signal transduction, cell death, and the like. A bioactive compound includes pharmaceutical compounds. In one embodiment, a 14-3-3 antibody is conjugated to a 14-3-3 antagonist peptide, preferably R-18, preferably via a linker.

### Peptides

In one aspect, the invention provides 14-3-3 antagonists that are peptides. Such peptides include CDR peptides.

In one embodiment, the peptide binds to a region of the 14-3-3 protein that is capable of binding to an anti-14-3-3 antibody or other 14-3-3 antagonist peptide. The term "peptide" or "oligopeptide" as used herein is meant to encompass peptide analogs, derivatives, fusion proteins and the like, as well as peptide compositions, including those exemplified in the present disclosure.

In a preferred embodiment, the peptide comprises the amino acid sequence designated "R-18". In another preferred embodiment, the peptide consists essentially of the R-18 sequence. In another preferred embodiment, the peptide comprises a segment of the R-18 sequence. In another preferred embodiment, the peptide comprises multiple iterations of the R-18 sequence, preferably separated by a linker.

In another preferred embodiment, the peptide binds to a region of a 14-3-3 protein that is capable of binding to R-18. In another preferred embodiment, the peptide binds to a region of a 14-3-3 protein that is capable of binding to an intracellular 14-3-3 binding partner, preferably Raf.

In one embodiment, the peptide binds to a 14-3-3 protein without disrupting binding of the 14-3-3 protein to an intracellular 14-3-3 binding partner.

In one embodiment, the 14-3-3 antagonist is a phosphopeptide.

### Peptide Modifications

The subject peptides may be modified in a variety of conventional ways well known to the skilled artisan. Any number of modifications may be done to achieve a peptide having desired characteristics. What is required of a peptide of the invention is that it retain the ability to function as a 14-3-3 antagonist.

Examples of modifications include the following. The terminal amino group and/or carboxyl group of the peptide and/or amino acid side chains may be modified by alkylation, amidation, or acylation to provide esters, amides or substituted amino groups. Heteroatoms may be included in aliphatic modifying groups. This is done using conventional chemical synthetic methods. Other modifications include deamination of glutamyl and asparaginyl residues to the corresponding glutamyl and aspartyl residues, respectively; hydroxylation of proline and lysine; phosphorylation of hydroxyl groups of serine or threonine; and methylation of amino groups of lysine, arginine, and histidine side chains (see, for example, T. E. Creighton, Proteins: Structure and Molecular Properties, W.H. Freeman & Co. San Francisco, Calif., 1983).

In another aspect, one or both, usually one terminus of the peptide, may be substituted with a lipophilic group, usually aliphatic or aralkyl group, which may include heteroatoms. Chains may be saturated or unsaturated. Conveniently, commercially available aliphatic fatty acids, alcohols and amines may be used, such as caprylic acid, capric acid, lauric acid, myristic acid and myristyl alcohol, palmitic acid, palmitoleic acid, stearic acid and stearyl amine, oleic acid, linoleic acid, docosahexaenoic acid, etc. (see U.S. Pat. No. 6,225,444). Preferred are unbranched, naturally occurring fatty acids between 14-22 carbon atoms in length. Other lipophilic molecules include glyceryl lipids and sterols, such as cholesterol. The lipophilic groups may be reacted with the appropriate functional group on the oligopeptide in accordance with conventional methods, frequently during the synthesis on a support, depending on the site of attachment of the oligopeptide to the support. Lipid attachment is useful where oligopeptides may be introduced into the lumen of the liposome, along with other therapeutic agents for administering the peptides and agents into a host.

In additional embodiments, either or both the N- and C-terminus of the peptide may be extended by not more than a total of about 100, usually not more than a total of about 30, more usually not more than about 20 amino acids, often not more than about 9 amino acids, where the amino acids will have fewer than 25%, more usually fewer than 20% polar amino acids, more particularly, fewer than 20% which are charged amino acids. Thus, extensions of the above sequences in either direction are mainly done with lipophilic, uncharged amino acids, particularly non-polar aliphatic amino acids and aromatic amino acids. The peptides may comprise L-amino acids, D-amino acids, or mixtures of D- and L-amino acids. Exceptions to the number of amino acid extensions are contemplated when the oligopeptides are expressed as fusion or chimeric proteins, as described below.

The peptides may also be in the form of oligomers, especially dimers of the peptides, which may be head to head, tail to tail, or head to tail, preferably with not more than about 6 repeats of the peptide. The oligomer may contain one or more D-stereoisomer amino acids, up to all of the amino acids. The oligomers may or may not include linker sequences between the peptides. Suitable linkers include, but are not limited to, those comprising uncharged amino acids and (Gly)n, where n is 1-7, Gly-Ser (e.g., (GS)ₙ, (GSGGS)ₙ and (GGGS)ₙ, where n is at least 1), Gly-Ala, Ala-Ser, or other flexible linkers, as known in the art. Linkers of Gly or Gly-Ser may be used since these amino acids are relatively unstructured, which allows interaction of individual peptides with cellular target molecules and limits structural perturbations between peptides of the oligomer. It is to be understood that linkers other than amino acids may be used to construct the oligomeric peptides.

Peptides may also be in a structurally constrained form, such as cyclic peptides, preferably of from about 9-50, usually 12 to 36 amino acids, where amino acids other than the specified amino acids may be present as a bridge. Thus, for example, addition of terminal cysteines allows formation of disulfide bridges to form a ring peptide. In some instances, one may use other than amino acids to cyclize the peptide. Bifunctional crosslinking agents are useful in linking two or more amino acids of the peptide. Other methods for ring formation are known in the art, see for example Chen, S. et al., Proc. Natl. Acad. Sci. USA 89:5872-5876 (1992); Wu, T. P. et al., Protein Engineering 6:471478 (1993); Anwer, M. K. et al., Int. J. Pep. Protein Res. 36:392-399 (1990); and Rivera-Baeza, C. et al. Neuropeptides 30: 327-333 (1996). Alternatively, structurally constrained peptides are made by addition of dimerization sequences to the N- and C-terminal ends of the peptide, where interaction between dimerization sequences lead to formation of a cyclic type structure (see, e.g., WO/0166565). In other instances, the subject peptides are expressed as fusions to other proteins, which provide a scaffold for constrained display on a surface exposed structure, such as a loop of a coiled-coil or β-turn structure.

Depending upon their intended use, particularly for administration to mammalian hosts, the subject peptides may also be modified by attachment to other compounds for the purposes of incorporation into carrier molecules, changing peptide bioavailability, extending or shortening half-life, controling distribution to various tissues or the blood stream, diminishing or enhancing binding to blood components, and the like. The subject peptides may be bound to these other components by linkers which are cleavable or non-cleavable in the physiological environment, e.g., by MMPs in the synovium. The peptides may be joined at any point of the peptide where a functional group is present, such as hydroxyl, thiol, carboxyl, amino, or the like. Desirably, modification will be at either the N-terminus or the C-terminus. For instance, the subject peptides may be modified by covalently attaching polymers, such as polyethylene glycol, polypropylene glycol, carboxymnethyl cellulose, dextran, polyvinyl alcohol, polyvinylpyrrolidine, polyproline, poly(divinyl-ether-co-malelc anhydride), poly(styrene-c-maleic anhydride), etc. Water-soluble polymers, such a polyethylene glycol and polyvinylpyrrolidine are known to decrease clearance of attached compounds from the blood stream as compared to unmodified compounds. The modifications can also increase solubility in aqueous media and reduce aggregation of the peptides. What is required is that the peptide, when delivered and/or released, retains 14-3-3 antagonist function.

### Peptide Conjugates and Fusion Proteins

In one embodiment, the peptide is conjugated to small molecules for detection and isolation of the peptides, or to target or transport the oligopeptide to specific cells, tissues, or organs. Small molecule conjugates include haptens, which are substances that do not initiate an immune response when introduced by themselves into an animal. Generally, haptens are small molecules of molecular weight less than about 2 kD, and more preferably less that about 1 kD. Haptens include small organic molecules (e.g., p-nitrophenol, digoxin, heroin, cocaine, morphine, mescaline, lysergic acid, tetrahydrocannabinol, cannabinol, steroids, pentamidine, biotin, etc.). Binding to the hapten, for example for purposes of detection or purification, are done with hapten specific antibodies or specific binding partners, such as avidin which binds biotin.

Small molecules that target the conjugate to specific cells or tissues may also be used.

It will be understood that labels well known in the art may also be attached to the peptides of the invention for use of such conjugates in diagnostic methods.

In one embodiment, the peptides are joined to any of a wide variety of other peptides or proteins for a variety of purposes. The peptides may be linked to peptides or proteins to provide convenient functionalities for bonding, such as amino groups for amide or substituted amine formation, e.g., reductive amination; thiol groups for thioether or disulfide formation; carboxyl groups for amide formation; and the like. Of particular interest are peptides of at least 2, more usually 3, and not more than about 60 lysine groups, particularly polylysines of from about 4 to 20, usually 6 to 18 lysine units, referred to as multiple antigenic peptide system (MAPS), where the subject peptides are bonded to the lysine amino groups, generally at least about 20%, more usually at least about 50%, of available amino groups, to provide a multipeptide product (Butz, S. et al., Pept. Res. 7: 20-23 (1994)). In this way, molecules having a plurality of the subject peptides are obtained where the orientation of the subject peptides is in the same direction; in effect, this linking group provides for tail-to-tail di- or oligomerization.

In one embodiment, the peptides are conjugated to other peptides or proteins for targeting the oligopeptide to cells and tissues, or adding additional functionalities to the peptides. For targeting, the protein or peptide used for conjugation will be selected based on the cell or tissue being targeted for therapy (Lee, R. et al., Arthritis. Rheum. 46: 2109-2120 (2002); Pasqualini, R., Q. J. Nucl. Med. 43: 159-62 (1999); Pasgualinl, R., Nature 380: 364-366 (1996)). The proteins may also compromise poly-amino acids including, but not limited to, polyarginine; and polylysine, polyaspartic acid, etc., which may be incorporated into other polymers, such as polyethylene glycol, for preparation of vesicles or particles containing the conjugated peptides.

In one embodiment, the subject peptides may be expressed or synthesized in conjunction with other peptides or proteins, to be a portion of the polypeptide chain, either internal, or at the N- or C- terminus to form chimeric proteins or fusion proteins. By "fusion polypeptide" or "fusion protein" or "chimeric protein" herein is meant a protein composed of a plurality of protein components that, while typically joined in the native state, are joined by the respective amino and carboxy termini through a peptide linkage to form a continuous polypeptide. It will be appreciated that the protein components can be joined directly or joined through a peptide linker/spacer.

Fusion polypeptides may be made to a variety of peptides or proteins to display the subject oligopeptides in a conformationally restricted form, for targeting to cells and tissues, for targeting to intracellular compartments, tracking the fusion protein in a cell or an organism, and screening for other molecules that bind the oligopeptides. Proteins useful for generating fusion proteins include various reporter proteins, structural proteins, cell surface receptors, receptor ligands, toxins, and enzymes. Exemplary proteins include fluorescent proteins (e.g., Aequodia victoria GFP, Renilla renifornis GFP, Renilla muelledi GFP, luciferases, etc., and variants thereof); β-galactosidase; alkaline phosphatase; E. coli. maltose binding protein; coat proteins of filamentous bacteriophage; T cell receptor; charybdotoxin; and the like.

Fusion proteins also encompass fusions with fragments of proteins or other peptides, either alone or as part of a larger protein sequence. Thus, the fusion polypeptides may comprise fusion partners. By "fusion partners" herein is meant a sequence that is associated with the peptide that confers all members of the proteins in that class a common function or ability. Fusion partners can be heterologous (i.e., not native to the host cell) or synthetic (ie., not native to any cell). The fusion partners include, but are not limited to, a) presentation structures, which provide the oligopeptides in a conformationally restricted or stable form; b) targeting sequences, which allow localization of the peptide to a subcellular or extracellular compartment; c) stability sequences, which affects stability or protection from degradation to the peptide or the nucleic acid encoding it; d) linker sequences, which conformationally decouples the oligopeptide from the fusion partner; and e) any combination of the above.

In one aspect, the fusion partner is a presentation structure. By "presentation structure" as used herein is meant a sequence that when fused to the subject peptides presents the peptides in a conformationally restricted form. Preferred presentation structures enhance binding interactions with other binding partners by presenting a peptide on a solvent exposed exterior surface. Generally, such presentation structures comprise a first portion joined to the N-terminus of the oligopeptide and a second portion joined to the C-terminal end of the oligopeptide. That is, the peptide of the present invention is inserted into the presentation structures. Preferably, the presentation structures are selected or designed to have minimal biological activity when expressed in the target cells.

Preferably, the presentation structures maximize accessibility to the peptides by displaying or presenting the peptide or an exterior loop. Suitable presentation structures include, but are not limited to, coiled coil stem structures, minibody structures, loops on β-turns, dimerization sequences, cysteine linked structures, transglutaminase linked structures, cyclic peptides, helical barrels, leucine zipper motifs, etc.

In one embodiment, the presentation structure is a coiled-coil structure, which allows presentation of the subject peptide on an exterior loop (e.g., Myszka, D. G. et al., Biochemistry 33: 2363-2373 (1994)), such as a coiled-coil leucine zipper domain (Martin, F. et al., EMBO J. 13: 5303-5309 (1994)). The presentation structure may also comprise minibody structures, which is essentially comprised of a minimal antibody complementary region. The minibody structure generally provides two peptide regions that are presented along a single face of the tertiary structure in the folded protein (e.g., Bianchi, E. et al., J. Mol. Biol. 236: 649-659 (1994); Tramontano, A. et al., J. Mol. Recognit. 7: 9-24 (1994)).

In another aspect, the presentation structure comprises two dimerization sequences. The dimerization sequences, which can be same or different, associate non-covalently with sufficient affinity under physiological conditions to structurally constrain the displayed peptide. Thus, if a dimerization sequence is used at each terminus of the subject oligopeptide, the resulting structure can display the subject peptide in a structurally limited or constrained form. A variety of sequences are suitable as dimerization sequences (see for example, WO 99/51625; incorporated by reference). Any number of protein-protein interaction sequences known in the art are useful for present purposes.

In a further aspect, the presentation sequence confers the ability to bind metal ions to generate a conformationally restricted secondary structure. Thus, for example, C2H2 zinc finger sequences are used. C2H2 sequences have two cysteines and two histidines placed such that a zinc ion is chelated. Zinc finger domains are known to occur independently in multiple zinc-finger peptides to form structurally independent, flexibly linked domains (e.g., Nakaseko, Y. et al., J. Mol. Biol. 228: 619-636 (1992)). A general consensus sequence is (5 amino acids)-C-(2 to 3 amino acids)-C-(4 to 12 amino acids)-H-(3 amino acids)-H-(5 amino acids) (SEQ ID NO:66). A preferred example would be -FQCEEC-random peptide of 3 to 20 amino acids-HIRSHTG (SEQ ID NO:67). Similarly, CCHC boxes having a consensus sequence -C-(2 amino acids)-C-(4 to 20 random peptide)-H-(4 amino acids)-C- (SEQ ID NO:68) can be used, (Bavoso, A. et al., Biochem. Biophys. Res. Commun. 242: 385389 (1998)). Other examples include (1)-VKCFNC-4 to 20 random amino acids-HTARNCR- (SEQ ID NO: 69), based on the nucleocapsid protein P2; (2) a sequence modified from that of the naturally occurring zinc-binding peptide of the Lasp-1 LIM domain (Hammarstrom, A. et al., Biochemistry 35: 12723-32 (1996)); and (3) -MNPNCARCG-4 to 20 random amino acids-HKACF- (SEQ ID NO:70), based on the NMR structural ensemble IZFP (Hammarstrom et al., supra).

In yet another aspect, the presentation structure is a sequence that comprises two or more cysteine residues, such that a disulfide bond may be formed, resulting in a conformationally constrained structure. That is, use of cysteine containing peptide sequences at each terminus of the subject oligopeptides results in cyclic peptide structures, as described above. A cyclic structure reduces susceptibility of the presented peptide to proteolysis and increases accessibility to its target molecules. As will be appreciated by those skilled in the art, this particular embodiment is particularly suited when secretory targeting sequences are used to direct the peptide to the extracellular space. In addition, sequences that are recognized and cleaved by proteases, such as the matrix metalloproteases (e.g., MMP-1, MMP-3), may be used. These residues are used to form circular peptides to increase peptide half-life or membrane permeability. Subsequent cleavage of the circular peptide with the appropriate protease releases the active, linear form of the peptide at the desired location.

In another embodiment, the fusion partner is a targeting sequence. Targeting sequences comprise binding sequences capable of causing binding of the expressed product to a predetermined molecule or class of molecules while retaining bioactivity of the expression product; sequences signaling selective degradation of the fusion protein or binding partners; and sequences capable of constitutively localizing peptides to a predetermined cellular locale. Typical cellular locations include subcellular locations (e.g., Golgi, endoplasmic recticulum, nucleus, nucleoli, nuclear membrane, mitochondria, secretory vesicles, lysosomes) and extracellular locations by use of secretory signals.

Various targeting sequences are known in the art, including membrane-anchoring sequences. Peptides are directed to the membrane via signal sequences and stably incorporated in the membrane through a hydrophobic transmembrane domain (designated as TM). The TM segment is positioned appropriately on the expressed fusion protein to display the subject peptide either intracellularly or extracellularly, as is known in the art. Especially preferred is extracellular presentation. Membrane anchoring sequences and signal sequences include, but are not limited to, those derived from (a) class I integral membrane proteins such as IL-2 receptor β-chain; Hatakeyama, M. et al., Science 244: 551-556 (1989)) and insulin receptor β-chain (Hetakeyama et al, supra); (b) class II integral membrane proteins such as neutral endopeptidase (Malfroy, B. et al Biochem. Biophys. Res. Commun. 144: 59-66 (1987)); and (c) type III proteins such as human cytochrome P450 NF25 (Hetakeyama et al, supra); and those from CD8, ICAM-2, IL-8R, and LFA-1.

Membrane anchoring sequences also include the GPI anchor, which results in covalent bond formation between the GPI anchor sequence and the lipid bilayer via a glycosyl-phosphatidylinositol. GPI anchor sequences are found in various proteins, including Thy-1 and DAF (Homans, S. W. et al., Nature 333: 269-272 (1988)). Similarly, acylation sequences allow for attachment of lipid moieties, e.g., isoprenylation (ie., famesyl and geranyl-geranyl; see Farnsworth, C. C. et al., Proc. Natl. Aced. Sci. USA 91: 11963-11967 (1994) and Aronheim, A. et al., Cell 78: 949-61 (1994)), myristoylation (Stickney, J. T. Methods Enzymol. 332: 64-77 (2001)), or palmitoylation. In one aspect, the subject peptide will be bound to a lipid group at a terminus, so as to be able to be bound to a lipid membrane, such as a liposome.

In another aspect, the targeting sequence is a secretory signal sequence which effects secretion of the peptide. A large number of secretory sequences are known to direct secretion of a peptide into the extracellular space when placed at the amino end relative to the peptide of interest, particularly for secretion of a peptide by cells, including transplanted cells. Suitable secretory signals included those found in IL-2 (Villinger, F. et al., J. immune. 155: 3946-3954 (1995)), growth hormone (Roskam, W. G. et al., Nucleic Acids Res. 7: 305-320 (1979)), preproinsulin, and influenza HA protein.

The fusion partner may further comprise a stability sequence, which confers stability to the fusion protein or the nucleic acid encoding it. Thus, for example, incorporation of glycines after the initiating methionine (e.g., MG or MGG) can stabilize or protect the fused peptide from degradation via ubiquitination as per the N-End rule of Varshavsky, thus conferring increased half-life in a cell.

Additional amino acids may be added for tagging the peptide for purposes of detection or purification. These sequences may comprise epitopes recognized by antibodies or sequences that bind ligands, such a metals ions. Various tag sequences and ligand binding sequences are well known in the art. These include, but is not limited to, poly-histidine (e.g., 6xHis tags, which are recognized by antibodies but also bind divalent metal ions); poly-histidine-glycine (poly-his-gly) tags; flu HA tag polypeptide; c-myc tag; Flag peptide (e.g., Hopp et al., BioTechnology 6: 1204-1210 (1988)); KT3 epitope peptide; tubulin epitope peptide (e.g., Skinner et al., J. Biol. Chem. 266: 15163-12166 (1991)); and T7 gene 10 protein peptide tag (e.g., Lutz-Freyermuth et al., Proc. Natl. Acad. Sci. USA 87: 6363-6397 (1990)).

Fusion partners include linker or tethering sequences, as discussed herein, for linking the peptides and for presenting the peptides in an unhindered structure.

In the present invention, combinations of fusion partners may be used. Any number of combinations of presentation structures, targeting sequences, tag sequences and stability sequences may be used with or without linker sequences.

### Peptide Preparation and Salts

The peptides of the invention may be prepared in a number of ways. Chemical synthesis of peptides is well known in the art. Solid phase synthesis is commonly used and various commercial synthetic apparatuses are available, for example automated synthesizers by Applied Biosystems Inc., Foster City, Calif.; Beckman; etc. Solution phase synthetic methods may also be used, particularly for large-scale productions. By using these standard techniques, naturally occurring amino acids may be substituted with unnatural amino acids, particularly D-stereoisomers, and with amino acids with side chains having different lengths or functionalities. Functional groups for conjugating to small molecules, label moieties, peptides, or proteins, or for purposes of forming cyclized peptides may be introduced into the molecule during chemical synthesis. In addition, small molecules and label moieties may be attached during the synthetic process. Preferably, introduction of the functional groups and conjugation to other molecules minimally affects the structure and function of the subject peptide.

The peptides of the present invention may also be present in the form of a salt, generally in a salt form which is pharmaceutically acceptable. These include inorganic salts of sodium, potassium, lithium, ammonium, calcium, magnesium, iron, zinc, copper, manganese, and the like. Various organic salts of the peptide may also be made with, including, but not limited to, acetic acid, propionic acid, pyruvic acid, maleic acid, succinic acid, tartaric acid, citric acid, benozic acid, cinnamic acid, salicylic acid, etc.

Synthesis of the oligopeptides and derivatives thereof may also be carried out by using recombinant techniques. For recombinant production, a nucleic acid sequence may be made which encodes a single oligopeptide or preferably a plurality of the subject peptides in tandem with an intervening amino acid or sequence, which allows for cleavage to the single peptide or head to tail dimers. Where methionine or tryptophane is absent, an intervening methionine or tryptophane may be incorporated, which allows for single amino acid cleavage using CNBr or BNPS-Skatole (2-(2-nitrophenylsulfenyl)-3-methyl-3-bromoindolenine), respectively. Alternatively, cleavage is accomplished by use of sequences that are recognized by particular proteases for enzymatic cleavage or sequences that act as self-cleaving sites (e.g., 2A sequences of apthoviruses and cardioviruses; Donnelly, M. L., J. Gen. Virol. 78:13-21 (1997); Donnelly, M. L., J. Gen. Virol. 82:1027-41 (2001)). The subject peptide may also be made as part of a larger peptide, which can be isolated and the oligopeptide obtained by proteolytic cleavage or chemical cleavage. The particular sequence and the manner of preparation will be determined by convenience, economics, purity required, and the like. To prepare these compositions, a gene encoding a particular peptide, protein, or fusion protein is joined to a DNA sequence encoding the oligopeptides of the present invention to form a fusion nucleic acid, which is introduced into an expression vector. Expression of the fusion nucleic acid is under the control of a suitable promoter and other control sequences, as defined below, for expression in a particular host cell or organism (Sambrook et al., Molecular Biology: A Laboratory Manual, Cold Spring Harbor Press, Cold Spring Harbor, N.Y. (3rd ed. 2001); Ausubel, F. et al., Current Protocols in Molecular Biology, John Wiley & Sons, New York, N.Y., (updates up to 2002) (1988)).

For conjugating various molecules to the peptides of the present invention, functional groups on the oligopeptides and the other molecule are reacted in presence of an appropriate conjugating (e.g., crosslinking) agent. The type of conjugating or crosslinking agent used will depend on the functional groups, such as primary amines, sulfhydryls, carbonyls, carbohydrates and carboxylic acids being used. Preferably, reactive functional groups on the oligopeptide not selected for modification are protected prior to coupling of the peptide to other reactive molecules to limit undesired side reactions. By "protecting group" as used herein is a molecule bound to a specific functional group which is selectively removable to reexpose the functional group (Greene, T. W. and Wuts, P. G. M. Protective Groups in Organic Synthesis, John Wiley & Sons, Inc., New York (3ed. 1999)). The peptides may be synthesized with protected amino acid precursors or reacted with protecting groups following synthesis but before reacting with crosslinking agent. Conjugations may also be indirect, for example by attaching a biotin moiety, which can be contacted with a compound or molecule which is coupled to streptavidin or avidin.

For oligopeptides that have reduced activity in the conjugated form, in a preferred embodiment, the linkage between the oliogopeptides and the conjugated compound is chosen to be sufficiently labile to result in cleavage under desired conditions, for example after transport to desired cells or tissues. Biologically labile covalent bonds, e.g., Imimo bonds and esters, are well known in the art (see, e.g., U.S. Pat. No. 5,108,921). These modifications permit administration of the oligopeptides in potentially a less active form, which is then activated by cleavage of the labile bond.

### Nucleic Acids, Expression Vectors, and Methods of Introduction

14-3-3 antagonists which are proteins, including peptides and antibodies, may be synthesized using nucleic acids encoding the same. This may be done to produce 14-3-3 antagonists which are subsequently isolated for use. Alternatively, such nucleic acids may be used therapeutically.

In one embodiment, the nucleic acids are cloned into expression vectors and introduced into cells or a host. The expression vectors are either self-replicating extrachromosomal vectors or vectors that integrate into the host chromosome, for example vectors based on retroviruses, vectors with site specific recombination sequences, or by homologous recombination. Generally, these vectors include control sequences operably linked to the nucleic acids encoding the oligopeptides. By "control sequences" is meant nucleic acid sequences necessary for expression of the subject peptides in a particular host organism. Thus, control sequences include sequences required for transcription and translation of the nucleic acids, including, but not limited to, promoter sequences, enhancer or transcriptional activator sequences, ribosomal binding sites, transcriptional start and stop sequences; polyadenylation signals; etc.

In a preferred embodiment, the cell is a fibroblast or FLS cell. Preferably the cells is a synovial cell. Preferably the cell is engineered to express a 14-3-3 antagonist. The cell may be manipulated in vitro and introduced into a recipient. Alternatively, the cell may be manipulated in vivo.

A variety of promoters are useful in expressing the peptides of the present invention. The promoters may be constitutive, inducible, and/or cell specific, and may comprise natural promoters, synthetic promoters (e.g., tTA tetracycline inducible promoters), or hybrids of various promoters. Promoters are chosen based on, among other considerations, the cell or organism in which the proteins are to be expressed, the level of desired expression, and any desired regulation of expression. Suitable promoters are bacterial promoters (e.g., pL1 phage promoter, tac promoter, lac promoter, etc.); yeast based promoters (e.g., GAL4 promoter, alcohol dehydrogenase promoter, tryptophane synthase promoter, copper inducible CUPI promoter, etc.), plant promoters (e.g., CaMV S35, nopoline synthase promoter, tobacco mosaic virus promoter, etc), insect promoters (e.g., Autographa nuclear polyhedrosis virus, Aedes DNV viral p& and p61, hsp70, etc.), and promoters for expression mammalian cells (e.g., ubiquitin gene promoter, ribosomal gene promoter, β-globin promoter, thymidine kinase promoter, heat shock protein promoters, and ribosomal gene promoters, etc.), and particularly viral promoters, such as cytomegalovirus (CMV) promoter, simian virus (SV40) promoter, and retroviral promoters.

By "operably linked" herein is meant that a nucleic acid is placed into a functional relationship with another nucleic acid. In the present context, operably linked means that the control sequences are positioned relative to the nucleic acid sequence encoding the subject 14-3-3 antagonists in such a manner that expression of the encoded antagonist occurs. The vectors may comprise plasmids or comprise viral vectors, for example retroviral vectors, which are useful delivery systems if the cells are dividing cells, or lentiviral and adenoviral vectors if the cells are non-dividing cells. Particularly preferred are self-inactivating retroviral vectors (SIN vectors), which have inactivated viral promoters at the 3'-LTR, thereby permitting control of expression of heterologous genes by use of non-viral promoters inserted into the viral vector (see, e.g., Hofmann, A. et al., Proc. Natl. Acad. Sci. USA 93: 5185-5190 (1996)). As will be appreciated by those in the art, modifications of the system by pseudotyping allows use of retroviral vectors for all eukaryotic cells, particularly for higher eukaryotes (Morgan, R. A. et al., J. Virol. 67: 4712-4721 (1993); Yang, Y. et al., Hum. Gene Ther. 6:1203-1213 (1995)).

In addition, the expression vectors also contain a selectable marker gene to allow selection of transformed host cells. Generally, the selection will confer a detectable phenotype that enriches for cells containing the expression vector and further permits differentiation between cells that express and do not express the selection gene. Selection genes are well known in the art and will vary with the host cell used. Suitable selection genes include genes that render the cell resistant to a drug, genes that permit growth in nutritionally deficient media, and reporter genes (e.g., β-galactosidase, fluorescent proteins, glucouronidase, etc.), all of which are well known in the art and available to the skilled artisan.

There are a variety of techniques available for introducing nucleic acids into viable cells. By "introduced" into herein is meant that the nucleic acid enters the cells in a manner suitable for subsequent expression of the nucleic acid. Techniques for introducing the nucleic acids will vary depending on whether the nucleic acid is transferred in vitro into cultured cells or in vivo into the cells of the intended host organism and will also depend on the type of host organism. Methods for introducing the nucleic acids in vitro include the use of liposomes, Lipofectin™, electroporation, microinjection, cell fusion, DEAE dextran, calcium phosphate precipitation, and biolistic particle bombardment. Techniques for transfer in vivo include direct introduction of the nucleic acid, use of viral vectors, typically retroviral vectors, and liposome mediated transfection, including viral coated liposome mediated transfection. The nucleic acids expressing the 14-3-3 antagonists of the present invention may exist transiently or stably in the cell or stably integrate into the chromosome of the host.

In some situations, it is desirable to include an agent that targets the target cells or tissues, such as an antibody specific for a cell surface protein or the target cell e.g., a fibroblast or FLS cell, a ligand for a receptor on the target cell, a lipid component on the cell membrane, or a carbohydrate on the cell surface. If liposomes are employed, proteins that bind a cell surface protein which is endocytosed may be used for targeting and/or facilitating uptake. These include as non-limiting examples, capsid proteins or fragments thereof tropic for a particular cell types, antibodies for proteins which undergo internalization (Wu, G. Y. et al., J. Biol. Chem. 262: 4429-4432 (1987); Wagner, E. et al., Proc. Natl. Aced. Sci. USA 87: 3410-3414 (1990)), or enhance in vivo half-life.

Expression is done in a wide range of host cells that span prokaryotes and eukaryotes, including bacteria, yeast, plants, insects, and animals. The oligopeptides of the present invention may be expressed in, among others, E. coli., Saccharomyces cerevislae, Saccharomyces pombe, Tobacco or Arabidopsis plants, insect Schneider cells, and mammalian cells, such as COS, CHO, HeLa, and the like, either intracellularly or in a secreted form by fusing the peptides to an appropriate signal peptide. Secretion from the host cell may be done by fusing the DNA encoding the oligopeptide and a DNA encoding a signal peptide. Secretory signals are well known in the art for bacteria, yeast, insects, plants, and mammalian systems. Nucleic acids expressing the oligopeptides may be inserted into cells, for example stem cells for tissue expression or bacteria for gut expression, and the cells transplanted into the host to provide an in vivo source of the oligopeptides.

### Purified Peptides

In a preferred embodiment, the oligopeptides of the present invention may be purified or isolated after synthesis or expression. By "purified" or "isolated" is meant free from the environment in which the peptide is synthesized or expressed and in a form where it can be practically used. Thus, by purified or isolated is meant that the peptide or its derivative is substantially pure, i.e., more than 90% pure, preferably more than 95% pure, and preferably more than 99% pure. The oligopeptides and derivatives thereof may be purified and isolated by methods known to those skilled in the art, depending on other components present in the sample. Standard purification methods include electrophoretic, immunological, and chromatographic techniques, including ion exchange, hydrophobic, affinity, size exclusion, reverse phase HPLC, and chromatofocusing. The proteins may also be purified by selective solubility, for instance in the presence of salts or organic solvents. The degree of purification necessary will vary depending on use of the subject oligopeptides. Thus, in some instances no purification will be necessary.

For most applications, the compositions used will comprise at least 20% by weight of the desired product, more usually at least about 75% by weight, preferably at least about 95% by weight, and usually at least about 99.5% by weight, relative to contaminants related to the method of product preparation, the purification procedure, and its intended use, for example with a pharmaceutical carrier for the purposes of therapeutic treatment. Usually, the percentages will be based upon total protein.

### Pharmaceutical Compositions, Administration, and Dosages

The 14-3-3 antagonists of the invention can be incorporated into pharmaceutical compositions suitable for administration to a subject. Typically, the pharmaceutical composition comprises a 14-3-3 antagonist of the invention and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable substances such as wetting or minor amounts of auxiliary substances such as wetting or emulsifying agents, preservatives or buffers, which enhance the shelf life or effectiveness of the 14-3-3 antagonist.

The 14-3-3 antagonists are targeted to 14-3-3 protein that is localized extracellularly. Accordingly, therapeutic compositions are formulated and administration is such that the 14-3-3 antagonist so delivered is available to engage extracellular 14-3-3 protein.

The compositions of this invention may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies. The preferred mode of administration is parenteral (e.g., intravenous, subcutaneous, intraperitoneal, intramuscular, with intracapsular being especially preferred). In one embodiment, the 14-3-3 antagonist is administered by intravenous infusion or injection. In another preferred embodiment, the 14-3-3 antagonist is administered by intramuscular or subcutaneous injection. In a preferred embodiment, direct injection into the synovium is done.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The 14-3-3 antagonists of the present invention can be administered by a variety of methods known in the art, including intravenous injection or infusion. Direct administration to the synovium is one preferred route of administration. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978. Representative formulation technology is taught in, inter alia, Remington: The Science and Practice of Pharmacy, 19th Ed., Mack Publishing Co., Easton, PA (1995) and Handbook of Pharmaceutical Excipients, 3rd Ed, Kibbe, A.H. ed., Washington DC, American Pharmaceutical Association (2000)

In certain embodiments, a 14-3-3 antagonist of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation.

Supplementary active compounds can also be incorporated into the compositions. In certain embodiments, a 14-3-3 antagonist of the invention is coformulated with and/or coadministered with one or more additional therapeutic agents. For example, a DMARD or DMOAD. Such combination therapies may advantageously utilize lower dosages of the administered therapeutic agents, thus avoiding possible toxicities or complications associated with the various monotherapies.

The pharmaceutical compositions of the invention may include a "therapeutically effective amount" or a "prophylactically effective amount" of an antibody of the invention. A "therapeutically effective amount" refers to an amount effective, at dosages and for periods of time necessary, to achieve the desired therapeutic result. A therapeutically effective amount of the 14-3-3 antagonist may vary according to factors such as the disease state, age, sex, and weight of the individual, and the ability of the 14-3-3 antagonist to elicit a desired response in the individual. A therapeutically effective amount is also one in which any toxic or detrimental effects of the antibody are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of an antibody of the invention is 0.1-20 mg/kg, more preferably 1-10 mg/kg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

The pharmaceutical compositions described herein may be presented in unit-dose or multi-dose containers, such as sealed ampoules or vials. Such containers are typically sealed in such a way to preserve the sterility and stability of the formulation until use. In general, formulations may be stored as suspensions, solutions or emulsions in oily or aqueous vehicles, as indicated above. Alternatively, a pharmaceutical composition may be stored in a freeze-dried condition requiring only the addition of a sterile liquid carrier immediately prior to use.

### Therapeutic Use of 14-3-3 Antagonists

By "treatment" herein is meant therapeutic or prophylactic treatment, or a suppressive measure for the disease, disorder or undesirable condition. Treatment encompasses administration of the subject 14-3-3 antagonists in an appropriate form prior to the onset of disease symptoms and/or after clinical manifestations, or other manifestations, of the disease to reduce disease severity, halt disease progression, or eliminate the disease. Prevention of the disease includes prolonging or delaying the onset of symptoms of the disorder or disease, preferably in a subject with increased susceptibility to the disease.

In one aspect, the invention provides methods of treating arthritis, including methods of treating ankylosing spondylitis, Behçet's Disease, diffuse idiopathic skeletal hyperostosis (DISH), Ehlers-Danlos Syndrome (EDS), Felty's Syndrome, fibromyalgia, gout, infectious arthritis, juvenile arthritis, lupus, mixed connective tissue disease (MCTD), osteoarthritis, Paget's Disease, polymyalgia rheumatica, polymyositis and dermatomyositis, pseudogout, psoriatic arthritis, Raynaud's Phenomenon, reactive arthritis, rheumatoid arthritis, scleroderma, Sjögren's Syndrome, Still's Disease, and Wegener's granulomatosis.

Generally, the methods comprise administering a 14-3-3 antagonist to a patient, either alone or in combination with other therapeutic agents to increase treatment efficacy.

### Methods of screening for 14-3-3 antagonists

In one aspect, the invention provides methods of screening for 14-3-3 antagonists. The compounds screened can range from small organic molecules to large polymers and biopolymers, and can include, by way of example and not limitation, small organic compounds, saccharides, carbohydrates, polysaccharides, lectins, peptides and analogs thereof, polypeptides, proteins, antibodies, oligonucleotides, polynucleotides, nucleic acids, etc.

In one embodiment, the candidate compounds screened are small organic molecules, preferably having a molecular weight in the range of about 100-2500 daltons, though other molecules may be used. Such candidate molecules will often comprise cyclical structures composed of carbon atoms or mixtures of carbon atoms and one or more heteroatoms and/or aromatic, polyaromatic, heteroaromatic and/or polyaromatic structures. The candidate agents may include a wide variety of functional group substituents. In one embodiment, the substituent(s) are independently selected from the group of substituents known to interact with proteins, such as, for example, amine, carbonyl, hydroxyl and carboxyl groups.

The candidate compounds may be screened on a compound-by-compound basis or, alternatively, using one of the myriad library techniques commonly employed in the art. For example, synthetic combinatorial compound libraries, natural products libraries and/or peptide libraries may be screened using the assays of the invention to identify compounds that compete with a 14-3-3 ligand for binding to a 14-3-3 protein. These competitive binding assays can identify compounds that bind the 14-3-3 protein at approximately the same site as the 14-3-3 ligand. Myriad techniques for carrying out competitive binding assays are known in the art. Any of these techniques may be employed in the present invention.

Such binding experiments may be conducted wholly in solution or, alternatively, using a solid support, e.g., a glass or other bead, or a solid surface such as, for example, the bottom of a petri dish, to immobilize a reagent. The immobilization may be mediated by non-covalent interactions or by covalent interactions. Methods for immobilizing myriad types of compounds and proteins on solid supports are well-known. Any of these methods may be used.

Whether carried out in solution or with an immobilized 14-3-3 protein or candidate compound, the 14-3-3 protein and candidate compound are typically contacted with one another under conditions conducive to binding. Although the actual conditions used can vary, typically the binding assays are carried out under physiological conditions. Actual concentrations suitable for a particular assay will be apparent to those of skill in the art.

In one embodiment, the assays further comprise functional assays for the ability of a candidate agent to antagonize 14-3-3 protein activity. In one embodiment, the assays comprise determining the ability of a candidate agent to reduce the induction of MMP by 14-3-3 protein. In one embodiment, candidate agent is mixed with 14-3-3 protein, and the mixture may be added to cells capable of inducing MMP in response to 14-3-3 protein. In another embodiment, candidate agent is added with 14-3-3 protein to cells capable of inducing MMP in response to 14-3-3 protein. Other functional assays measuring the ability of a candidate agent to inhibit the ability of a 14-3-3 protein to induce a measurable change in cells, preferably fibroblasts or FLS cells, and thus to characterize the candidate as a 14-3-3 antagonist, can be undertaken.

All citations herein, including those listed below, are expressly incorporated herein in their entirety by reference.

### EXPERIMENTAL

**Table 1: 14-3-3 Eta epitopes**

| | | | |
|---|---|---|---|
| SEQ ID NO:1 | 93-107 | helix | LETVCNDVLSLLDKF |
| SEQ ID NO:2 | 191-199 | helix | EQACLLAKQ |
| SEQ ID NO:3 | 144-155 | helix | NSVVEASEAAYK |
| SEQ ID NO:4 | 144-152 | helix | NSVVEASEA |
| SEQ ID NO:5 | 147-155 | helix | VEASEAAYK |
| SEQ ID NO:6 | 163-170 | helix | EQMQPTHP |
| SEQ ID NO:7 | 168-177 | helix | THPIRLGLAL |
| SEQ ID NO:8 | 82-92 | helix | VKAYTEKIEKE |
| SEQ ID NO:9 | 68-79 | helix | QKTMADGNEKKL |
| SEQ ID NO:10 | 138-146 | helix | ASGEKKNSV |
| SEQ ID NO:11 | 69-77 | loop | KTMADGNEK |
| SEQ ID NO:12 | 32-40 | loop | ELNEPLSNE |
| SEQ ID NO:13 | 103-117 | loop | LLDKFLIKNCNDFQY |
| SEQ ID NO:14 | 130-143 | loop | YYRYLAEVASGEKK |
| SEQ ID NO:15 | 184-194 | loop | YEIQNAPEQAC |
| SEQ ID NO:16 | 206-218 | loop | AELDTLNEDSYKD |
| SEQ ID NO:17 | 44-57 | non-helix | LLSVAYKNWGARR |
| SEQ ID NO:18 | 15-23 | non-helix | EQAERYDDM |
| SEQ ID NO:19 | 130-138 | non-helix | YYRYLAEVA |
| SEQ ID NO:20 | 118-125 | non-helix | ESKVFYLK |
| SEQ ID NO:21 | 210-218 | non-helix | TLNEDSYKD |
| SEQ ID NO:22 | 77-84 | non-helix | KKLEKVKA |
| SEQ ID NO:23 | 76-86 | non-helix | EKKLRKVKAYR |
| SEQ ID NO:24 | 142-158 | non-helix | KKNSVVEASEAAYKEAF |
| SEQ ID NO:25 | 105-120 | non-helix | DKFLIKNCNDFQYESK |
| SEQ ID NO:26 | 237-246 | non-helix | QQDEEAGEGN |
| SEQ ID NO:27 | 75-82 | non-helix | NEKKLEKVK |
| SEQ ID NO:28 | 104-116 | non-helix | LDKFLIKNCNDFQ |
| SEQ ID NO:29 | 141-146 | non-helix | EKKNSV |
| SEQ ID NO:30 | 104-115 | non-helix | LDKFLIKNS*NDF |
| SEQ ID NO:31 | 77-86 | non-helix | KKLEKVKAYR |
| SEQ ID NO:32 | 143-157 | non-helix | KNSVVEASEAAYKEA |
| SEQ ID NO:65 | 1-12 | non-helix | DREQLLQRARLA |

| | | | |
|---|---|---|---|
| *The internal cysteine amino acid was replaced by the amino acid serine to prevent formation of disulfide bonds. | | | |

**Table 2: 14-3-3 gamma epitopes**

| | | | |
|---|---|---|---|
| SEQ ID NO:33 | 93-107 | helix | LEAVCQDVLSLLDNY |
| SEQ ID NO:34 | 191-199 | helix | EQACHLAKT |
| SEQ ID NO:35 | 144-155 | helix | ATVVESSEKAYS |
| SEQ ID NO:36 | 144-152 | helix | ATVVESSEK |
| SEQ ID NO:37 | 147-155 | helix | VESSEKAYS |
| SEQ ID NO:38 | 168-177 | helix | THPIRLGLAL |
| SEQ ID NO:39 | 82-92 | helix | VRAYREKIEKE |
| SEQ ID NO:40 | 68-79 | helix | QKTSADGNEKKI |
| SEQ ID NO:41 | 138-146 | helix | ATGEKRATV |
| SEQ ID NO:42 | 163-170 | helix | EHMQPTHP |
| SEQ ID NO:43 | 141-146 | helix | EKRATV |
| SEQ ID NO:44 | 69-77 | loop | KTSADGNEK |
| SEQ ID NO:45 | 32-40 | loop | ELNEPLSNE |
| SEQ ID NO:46 | 103-117 | loop | LLDNYLIKNCSETQY |
| SEQ ID NO:47 | 130-143 | loop | YYRYLAEVATGEKR |
| SEQ ID NO:48 | 184-194 | loop | YEIQNAPEQAC |
| SEQ ID NO:49 | 206-218 | loop | AELDTLNEDSYKD |
| SEQ ID NO:50 | 44-57 | non-helix | LLSVAYKNVVGARR |
| SEQ ID NO:51 | 75-83 | non-helix | NEKKIEMVR |
| SEQ ID NO:52 | 15-23 | non-helix | EQAERYDDM |
| SEQ ID NO:53 | 130-138 | non-helix | YYRYLAEVA |
| SEQ ID NO:54 | 118-125 | non-helix | ESKVFYLK |
| SEQ ID NO:55 | 210-218 | non-helix | TLNEDSYKD |
| SEQ ID NO:56 | 77-84 | non-helix | KKIEMVRA |
| SEQ ID NO:57 | 76-86 | non-helix | EKKIEMVRAYR |
| SEQ ID NO:58 | 142-158 | non-helix | KRATWESSEKAYSEAH |
| SEQ ID NO:59 | 105-120 | non-helix | DNYLIKNCSETQYESK |
| SEQ ID NO:60 | 237-246 | non-helix | QQDDDGGEGN |
| SEQ ID NO:61 | 75-82 | non-helix | NEKKIEMV |
| SEQ ID NO:62 | 104-116 | non-helix | LDNYLIKNCSETQ |

**Table 3. Protein Sequences of recombinant human 14-3-3 eta (SEQ ID NO:63) and recombinant human 14-3-3 gamma (SEQ ID NO: 64)**

| | |
|---|---|
| SEQ ID NO:63 | MGDREQLLQR ARLAEQAERY DDMASAMKAV TELNEPLSNE 40 DRNLLSVAYK NVVGARRSSW RVISSIEQKT MADGNEKKLE 80 KVKAYREKIE KELETVCNDV LSLLDKFLIK NCNDFQYESK 120 VFYLKMKGDY YRYLAEVASG EKKNSVVEAS EAAYKEAFEI 160 SKEQMQPTHP IRLGLALNFS VFYYEIQNAP EQACLLAKQA 200 FDDAIAELDT LNEDSYKDST LIMQLLRDNL TLWTSDQQDE 240 EAGEGN |
| SEQ ID NO:64 | |

In sequences comprising a cysteine residue, in one embodiment, the cysteine residue is replaced by a serine residue to avoid the formation of disulfide bonds. The cysteine may be an internal cysteine residue or a terminal cysteine residue.

Peptide epitopes may be modified for various purposes, including conjugation to an additional moiety, e.g., conjugation to a moiety to produce an immunogen comprising the epitope. As will be appreciated, cysteine may be placed appropriately for conjugation to carrier and to provide for exposure of the area that is desired to be exposed for purposes of making antibody. In case of KKLE the cysteine was added on to the C-terminal end in order to expose the other side. The carrier used may be quite large and may mask the first few amino acids.

### Example 1: 14-3-3 eta Immunogen Sequences and anti-14-3-3 eta antibodies

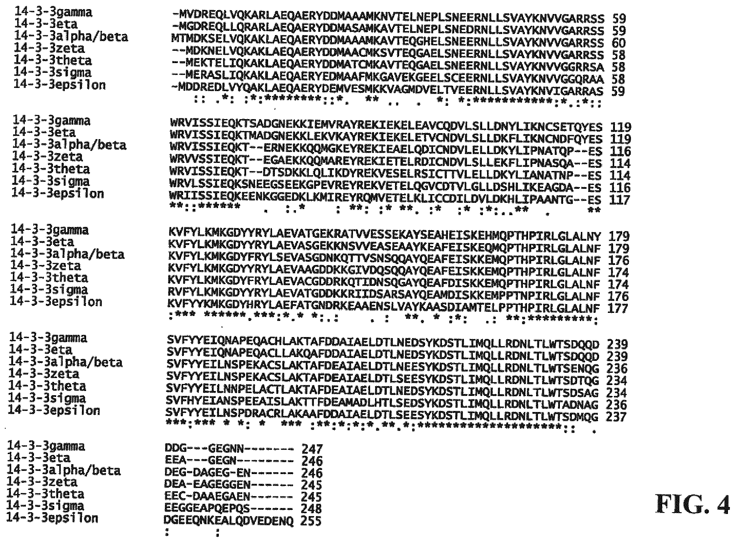

To prepare monospecific anti-14-3-3 eta antibodies, various peptides, 8 to 15 amino acids in length, were selected based on our own criteria. These peptides, as well as full-length recombinant native (untagged) 14-3-3 eta were used as immunogens in the production of monoclonal antibodies. A protein sequence alignment for the 7 isoforms of 14-3-3 is shown in Figure 4 (14-3-3 gamma (SEQ ID NO: 64, 14-3-3 eta (SEQ ID NO:63), 14-3-3 alpha/beta (SEQ ID NO: 71), 14-3-3 zeta (SEQ ID NO:72), 14-3-3 theta (SEQ ID NO:73), 14-3-3 sigma (SEQ ID NO:74), and 14-3-3 epsilon (SEQ ID NO:75)).

Immunogen #1: C-LDKFLIKNSNDF (SEQ ID NO:76) (Amino Acid Sequence 104 - 115; "AUG1-CLDK"). A peptide corresponding to a segment of human 14-3-3 eta residues 104-115 was modified by addition of an N-terminal cysteine moiety for conjugation to carrier, and replacement of internal cysteine-112 moiety to avoid formation of internal disulphide bonds.

Immunogen #2: KKLEKVKAYR-C (SEQ ID NO:77) (Amino Acid Sequence 77 - 86; "AUG2-KKLE"). A peptide corresponding to a segment of human 14-3-3 eta residues 77-86 was modified by addition of a C-terminal cysteine moiety for conjugation to carrier.

Immunogen #3: C-KNSVVEASEAAYKEA (SEQ ID NO:78) (Amino Acid Sequence 143 -157; "AUG3-CKNS"). A peptide corresponding to a segment of human 14-3-3 eta residues 143-157 was modified by addition of an N-terminal cysteine moiety for conjugation to carrier.

Immunogen #4: Full length human recombinant 14-3-3 eta (SEQ ID NO: 63), Protein Accession #: NP_003396.

### Immunization

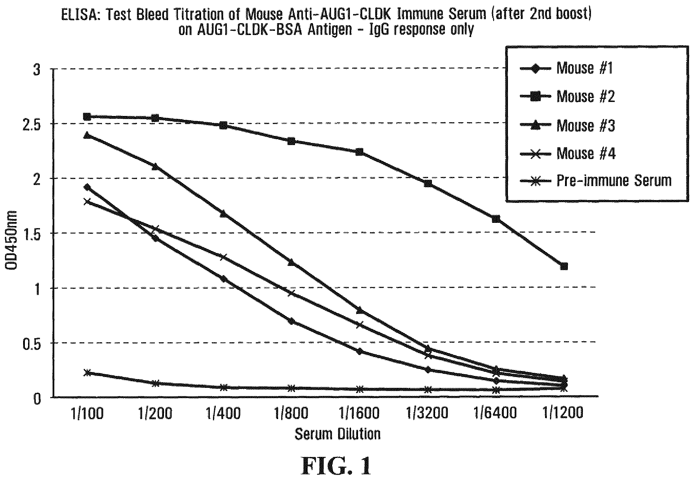

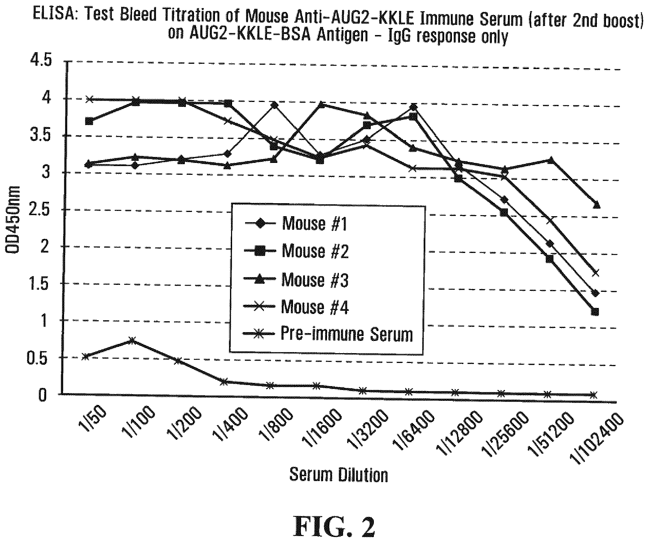

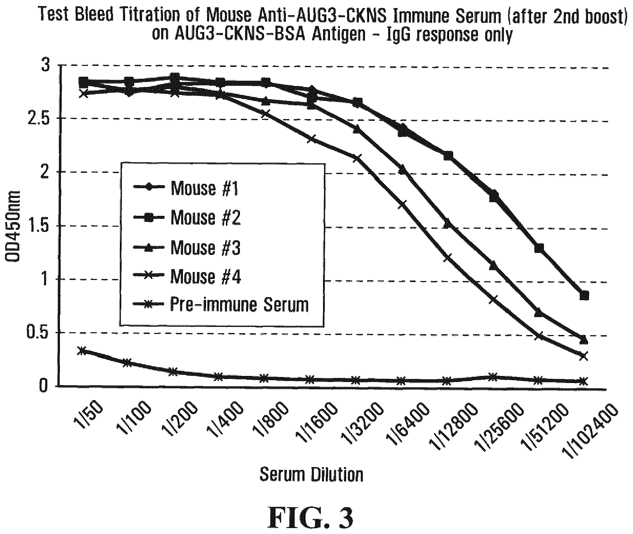

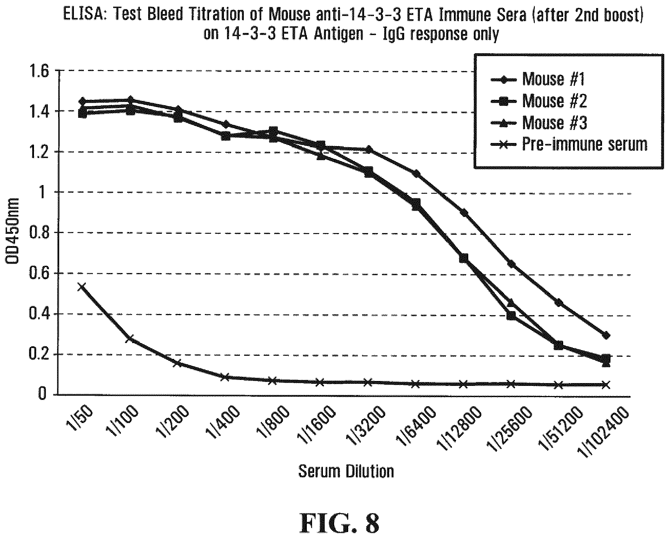

Groups of 4 female BALB/c mice were initially immunized by intraperitoneal injections using 50 ug of antigen (Immunogen #1, #2, #3 or #4) per mouse in Complete Freund's Adjuvant. Four subsequent boosts were administered as above, spaced at 3 week intervals, with antigen in Incomplete Freund's Adjuvant. When the serum titre had risen more than 10-fold from that of the pre-immune serum sample, as determined by ELISA, the 2 highest responders in each group were each boosted intravenously with 10 ug of antigen in 100 ul of sterile PBS pH 7.4. The titrations of serum samples from the immunized mice taken after the second boost are shown in Figure 1 (Immunogen #1; CLDK), Figure 2 (Immunogen #2; KKLE), Figure 3 (Immunogen #3; CKNS) and Figure 8 (Immunogen #4).

### Fusion Method

Three days after the final boost, the donor mice were sacrificed and the spleen cells were harvested and pooled. Fusion of the splenocytes with SP2/0 BALB/c parental myeloma cells was performed as previously described (Kohler et al., *infra*), except that one-step selection and cloning of the hybridomas was performed. Clones were picked 11 days post fusion and resuspended in wells of 96-well tissue culture plates in: 200 µl of D-MEM medium containing 1% hypoxanthine/thymidine, 20% fetal bovine serum, 2 mM GlutaMax I, 1 mM Sodium Pyruvate, 50 µg/ml Gentamycin, 1% OPI and 0.6 ng/ml IL-6. After 4 days, the supernatants were screened by ELISA for antibody activity on plates coated with 1 µg/well of purified antigen.

### Procedure for Revival of Slow Growing Hybridoma Clones

Hybridoma cell lines that were growing slowly or looked unhealthy could usually be rescued by the addition of a rich growth media containing: D-MEM medium with 1% hypoxanthine/thymidine, 20% fetal bovine serum, 2 mM GlutaMax I, 1 mM Sodium Pyruvate, 50 µg/ml Gentamycin, 1% OPI, 20% conditioned EL-4 tissue culture supernatant and 0.6 ng/ml IL-6. EL-4 is a murine thymoma cell line, which when stimulated with phorbal 12-myristate 12- acetate (PMA, from Sigma, cat # P-8139) causes the cells to secrete interleukin 2 (IL-2), a B cell differentiating factor (EL-BCDF-nak), and two B cell growth factors (BSF-p1 and EL-BCGF-swa) and other additional lymphokines, which greatly enhance lymphocyte growth and differentiation. See G. Kohler, and C. Milstein, Preparation of monoclonal antibodies, Nature 25 (1975) 256-259; Ma, M., S. Wu, M. Howard and A. Borkovec. 1984. Enhanced production of mouse hybridomas to picomoles of antigen using EL-4 conditioned media with an in vitro immunization protocol. In Vitro 20:739.
After 30 days of stability testing, a total of 100 viable clones were obtained that secreted IgG capable of recognizing recombinant 14-3-3 eta. For the purposes of identifying lead clones to pursue, the 100 viable clones were screened using a series of methods including: immunoblotting (dot blot), a trapping assay and a custom capture (sandwich) ELISA. All 100 clones were also tested for cross-reactivity using the custom capture (sandwich) ELISA with the other six 14-3-3 isoforms.

### Example 2 Testing the cross-reactivity of tissue culture (TC) supernatants from hybridoma clones using biotinylated 14-3-3 isoforms as bait in a capture ELISA

We have utilized a custom capture ELISA using the seven 14-3-3 isoforms as "bait" to determine whether any of the hybridoma clones that we have produced cross-react or recognize any of the six isoforms other than 14-3-3 Eta (η). As is evidenced by the representative data presented in Table 4, four of the selected hybridoma clones (AUG3-CKNS-2D5, AUG3-CKNS-7F8, AUG3-CKNS-7H8, AUG4-ETA-8F10) bind to and recognize 14-3-3 Eta at two serial dilutions, but do not bind with or cross-react with any of the other 14-3-3 isoforms, even at the lower dilution tested. This data clearly demonstrates that these clones are highly specific for 14-3-3 Eta (η). By contrast, one clone, AUG3-CKNS-4F10, binds with or cross-reacts with three other 14-3-3 isoforms, mainly 14-3-3 gamma, beta and zeta respectively. Taken together, these data indicate that our custom capture ELISA represents an effective method for screening and identifying hybridoma clones which are highly specific for the 14-3-3 Eta (η) isoform.

The Custom Capture ELISA experiment in Table 4 was carried out as follows. ELISA plates were coated with neat overgrown TC supernatant at 100µL/well and incubated overnight at 4°C. Biotin-labelled 14-3-3 (corresponding to all seven isoforms) was titrated from 1/500 to 1/16000 overtop and incubated for 1 hour at room temperature. Plates were then blocked with 3% skim milk powder in PBS (pH 7.4) at 100µL/well and incubated for 1 hour at room temperature. 1/8000 Streptavidin-HRPO was diluted in PBS-Tween, added at 100µL/well and incubated for 1 hour at 37°C with shaking. TMB buffer was added at 50µL per well and incubated in the dark at room temperature. Reactions were stopped with 50µL 1M HCl per well after 10 minutes and read at OD450nm.

**Table 4 a: Testing Cross-reactivity by ELISA**

| | **Testing the cross-reactivity of tissue culture (TC) supernatants from hybridoma clones using biotinylated 14-3-3 isoforms as bait in a capture ELISA (measured at OD₄₅₀nm)** | | | | | | | | | | | | | |
|---|---|---|---|---|---|---|---|---|---|---|---|---|---|---|
| **14-3-3 Isoform:** | **Gamma (γ)** | | **Beta (β)** | | **Sigma (σ)** | | **Theta/Tau (θ)** | | **Zeta (ζ)** | | **Epsilon (ε)** | | **Eta**□□□□ | |
| **dilution:** | **1:150 0** | **1:300 0** | **1:150 0** | **1:300 0** | **1:150 0** | **1:300 0** | **1:150 0** | **1:300 0** | **1:150 0** | **1:300 0** | **1:150 0** | **1:300 0** | **1:150 0** | **1:300 0** |
| **TC supernatant:** | | | | | | | | | | | | | | |
| **AUG3-CKNS-2D5** | 0.076 | 0.073 | 0.085 | 0.075 | 0.084 | 0.076 | 0.101 | 0.082 | 0.097 | 0.076 | 0.074 | 0.064 | **0.351** | **0.263** |
| ***AUG3-CKNS-4F10** | 0.084 | **0.076** | **0.096** | **0.085** | 0.125 | 0.102 | 0.185 | 0.153 | **0.167** | **0.122** | 0.139 | 0.101 | 0.114 | 0.09 |
| **AUG3-CKNS-7F8** | 0.072 | 0.067 | 0.078 | 0.076 | 0.083 | 0.076 | 0.116 | 0.104 | 0.093 | 0.084 | 0.089 | 0.076 | **0.946** | **0.741** |
| **AUG3-CKNS-7H8** | 0.07 | 0.066 | 0.072 | 0.063 | 0.087 | 0.078 | 0.098 | 0.083 | 0.089 | 0.08 | 0.074 | 0.064 | **0.774** | **0.608** |
| **AUG4-ETA-8F10** | 0.072 | 0.069 | 0.073 | 0.069 | 0.092 | 0.084 | 0.109 | 0.097 | 0.099 | 0.082 | 0.099 | 0.09 | **0.169** | **0.131** |
| **pre-immune serum (1:250)** | 0.097 | 0.074 | 0.093 | 0.081 | 0.136 | 0.113 | 0.193 | 0.158 | 0.152 | 0.119 | 0.144 | 0.115 | 0.152 | 0.11 |

| | | | | | | | | | | | | | | |
|---|---|---|---|---|---|---|---|---|---|---|---|---|---|---|
| **control antibody* | | | | | | | | | | | | | | |

**Table 4b: Testing Cross-reactivity by ELISA (background (pre-immune serum) values subtracted out)**

| | **Testing the cross-reactivity of tissue culture (TC) supernatants from hybridoma clones using biotinylated 14-3-3 isoforms as bait in a capture ELISA (measured at OD₄₅₀nm)** | | | | | | | | | | | | | |
|---|---|---|---|---|---|---|---|---|---|---|---|---|---|---|
| **14-3-3 Isoform:** | **Gamma (γ)** | | **Beta (β)** | | **Sigma (σ)** | | **Theta/Tau (θ)** | | **Zeta (ζ)** | | **Epsilon (ε)** | | **Eta**□□□□ | |
| **dilution:** | **1:150 0** | **1:300 0** | **1:150 0** | **1:300 0** | **1:150 0** | **1:300 0** | **1:150 0** | **1:300 0** | **1:150 0** | **1:300 0** | **1:150 0** | **1:300 0** | **1:150 0** | **1:300 0** |
| **TC supernatant:** | | | | | | | | | | | | | | |
| **AUG3-CKNS-2D5** | -0.021 | -0.001 | -0.008 | -0.006 | -0.052 | -0.037 | -0.092 | -0.076 | -0.055 | -0.043 | -0.070 | -0.051 | **0.199** | **0.153** |
| ***AUG3-CKNS-4F10** | -0.013 | **0.002** | **0.003** | **0.004** | -0.011 | -0.011 | -0.008 | -0.005 | **0.015** | **0.003** | -0.005 | -0.014 | -0.038 | -0.020 |
| **AUG3-CKNS-7F8** | -0.025 | -0.007 | -0.015 | -0.005 | -0.053 | -0.037 | -0.077 | -0.054 | -0.059 | -0.035 | -0.055 | -0.039 | **0.794** | **0.631** |
| **AUG3-CKNS-7H8** | -0.027 | -0.008 | -0.021 | -0.018 | -0.049 | -0.035 | -0.095 | -0.075 | -0.063 | -0.039 | -0.070 | -0.051 | **0.622** | **0.498** |
| **AUG4-ETA-8F10** | -0.025 | -0.005 | -0.020 | -0.012 | -0.044 | -0.029 | -0.084 | -0.061 | -0.053 | -0.037 | -0.045 | -0.025 | **0.017** | **0.021** |

| | | | | | | | | | | | | | | |
|---|---|---|---|---|---|---|---|---|---|---|---|---|---|---|
| **control antibody* | | | | | | | | | | | | | | |

### Example 3: 14-3-3 expression in synovial fluid and serum of RA affected patients

The levels of the different isoforms of 14-3-3 proteins - β, γ, ε, η, τ σ and ζ -in pooled patient synovial fluid (SF) and serum (PS) samples were analyzed by western analysis using keratinocyte cell lysate (K) as a positive control. Only the η and γ isoforms were detected in SF samples, and stained with greater intensity compared to PS. Articular joint synovial fluid samples from 17 RA patients who presented with active synovitis, but had not yet received anti-TNF therapies also exhibited consistent expression of the n isoform of 14-3-3 (data not shown). All patients had a disease activity score (DAS) greater than 6.0.

### Example 4: MMP expression in patient synovial fluid serum

To determine if these variations were correlated to those of MMP-1 and MMP-3 in the same synovial samples, a total of 12 RA synovial fluid samples and their matched serum samples were simultaneously evaluated for 14-3-3 n and γ as well as for MMP-1 and MMP-3 proteins. 14-3-3 n was detected in all samples. MMP-1 was detected in all samples, both SF and PS, while MMP-3 was more variable in the levels detected. The 14-3-3 γ isoform was also detected in patient synovial fluid and serum samples (data not shown).

The expression of MMP-1 and MMP-3 demonstrate significant correlation with the expression of the 14-3-3 n and γ isoforms in both synovial fluid and serum (Table 5).

**Table 5. Correlation of MMP and 14-3-3 protein levels in serum and synovial fluid.**

| | 14-3-3 η | 14-3-3 η | 14-3-3 γ | 14-3-3 γ |
|---|---|---|---|---|
| | serum | Synovium | serum | synovium |
| MMP-1 | r=0.62; p=0.02 | r=0.83; p=0.03 | r=0.77; p=0.02 | r=0.65; p=0.03 |
| MMP-3 | r=0.68; p=0.01 | r=0.77; p=0.003 | r=0.80; p=0.03 | r=0.76; p=0.04 |

### Example 5: Sensitivity of western blot detection of 14-3-3 protein in patient serum and synovial fluid samples.

To determine the detection level of 14-3-3 η in synovial fluid and serum samples, samples from 12 RA-affected or normal patients were pooled, and limiting dilutions of the pooled samples were analyzed by western blot. 14-3-3 η was detectable over a range of dilutions - as low as 0.1 µl effective volume of synovial fluid and 1.0 µl effective volume of serum (data not shown).

2 µl of pooled normal serum (NS) or patient serum (PS) was run alongside known concentrations of recombinant 14-3-3 η, ranging from 0.05 -2.0 µg. The 2 µl volume of NS and PS samples was estimated to have approximately 1-1.5 and 15-20 µg of 14- 3-3 η, respectively (data not shown). This suggests that the level of 14-3-3 η occurs in about a 10-fold excess in the serum of RA affected patients, compared to normal patients.

For more details, and results, see Kilani et al., J. Rheumatology, 34:1650-1657, 2007.

### Example 6: R-18 peptide interacts with extracellular 14-3-3 protein and inhibits induction of MMP-1 expression induced by extracellular 14-3-3 protein

The sequence of biotinylated R18 is as follows: Biotin-Pro-His-Cys-Val-Pro-Arg-Asp-Leu-Ser-Trp-Leu-Asp-Leu-Glu-Ala-Asn-Met-Cys-Leu-Pro-OH (SEQ ID NO:79).

In order to demonstrate the ability of R-18 to block or suppress the MMP-inducing effect of 14-3-3 proteins, we treated subconfluent cultures of dermal fibroblasts with keratinocyte-like cell conditioned medium (KLCCM) that has been demonstrated by mass spectrometry to contain several 14-3-3 isoforms (unpublished data). The KLCCM used in these experiments was taken from day 28 of cell transdifferentiation due to their high capacity of inducing MMP-1 expression in dermal fibroblasts (data not shown). Some samples of the conditioned media (KLCCM) were exposed to biotinylated R-18 and avidin sepharose to remove or "pull down" the 14-3-3 proteins present within the KLCCM.

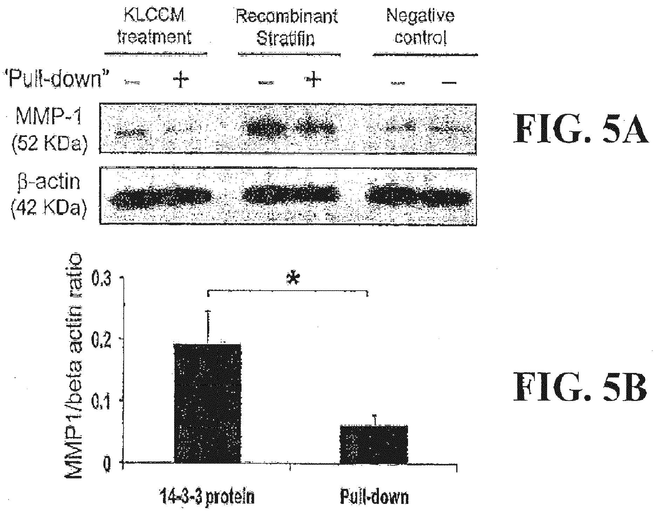

The results demonstrated that dermal fibroblasts expressed MMP-1, after treatment with the KLCCM and that the expression of MMP-1 could be partially inhibited (68.8% reduction) by pre-treatment of the KLCCM with biotinylated R-18 and avidin sepharose, which would selectively deplete the KLCCM of 14-3-3 proteins (Figure 5, Panel A "Pull-down).

Recombinant 14-3-3 sigma (stratifin), which is known to induce MMP-1, was used as positive control at 5 µg/ml ("Pulldown"-) or after exposure ("Pull-down"+). As negative control, dermal fibroblasts treated with medium that was not conditioned (DMEM (49%), KSFM (49%) plus 2% FBS) were used ("Pull-down" -). Panel B of Figure 5 shows the densitometric analysis of MMP-1/β-actin ratio. Findings from three independent experiments exhibited statistical significance (P value: 0.02).

### Example 7 Immunoprecipation of human recombinant 14-3-3 eta and endogenous 14-3-3 eta from HeLa cells

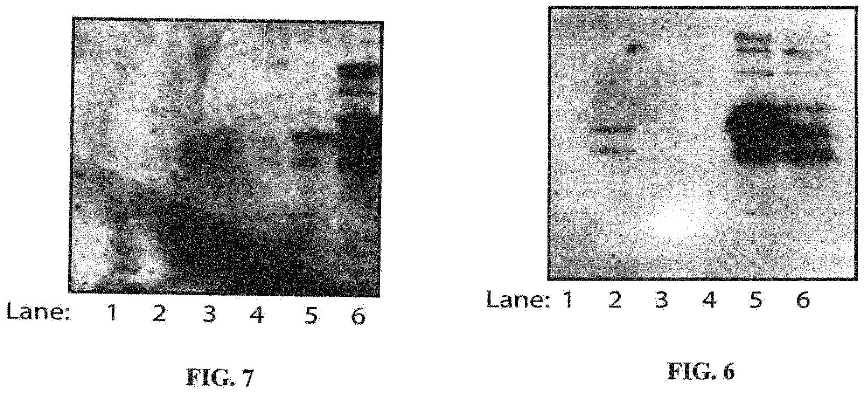

Monoclonal anti-14-3-3 antibodies from Example 1 were tested for their ability to immunoprecipitate or "capture" both recombinant and endogenous cellular 14-3-3 eta. For the therapeutic methods of the invention described herein, it is preferable to use antibodies that have the ability to immunoprecipitate or recognize 14-3-3 eta in its native 3-D configuration. Culture supernatants from anti 14-3-3 eta hybridoma clones were incubated at 4°C for 2 hours with either buffer containing 100 ng human recombinant 14-3-3 eta, or buffer containing supernatant (200 µg protein) from lysed HeLa cells. Immunoprecipitates were collected with Protein A/G agarose using standard methodology. Immunoprecipitates were analysed by SDS-PAGE and Western Blotting. Figure 6 shows a Western Blot obtained using Hybridoma clone **7B11**, which was made using Immunogen #4 (full length recombinant 14-3-3 eta. Lane 1: Protein A/G agarose beads alone; Lane 2: Protein A/G agarose beads were mixed with cell lysate; Lane 3: Protein A/G agarose beads were mixed with recombinant human 14-3-3 eta; Lane 4: Protein A/G agarose beads were mixed with hybridoma supernatant; Lane 5: Protein A/G agarose beads were mixed with hybridoma supernatant and cell lysate; Lane 6: Protein A/G agarose beads were mixed with hybridoma supernatant and recombinant 14-3-3 eta. The data show that clone **7B11** immunoprecipitated both HeLa cell-derived 14-3-3 eta (Lane 5) and human recombinant 14-3-3 eta (Lane 6).

Figure 7 shows a Western Blot obtained by using hybridoma clone **2D5** made against Immunogen #3 (CKNS). Lane 1: Protein A/G agarose beads alone; Lane 2: Protein A/G agarose beads were mixed with cell lysate; Lane 3: Protein A/G agarose beads were mixed with recombinant human 14-3-3 eta; Lane 4: Protein A/G agarose beads were mixed with hybridoma supernatant; Lane 5: Protein A/G agarose beads were mixed with hybridoma supernatant and cell lysate; Lane 6: Protein A/G agarose beads were mixed with hybridoma supernatant and recombinant 14-3-3 eta. The data show that clone **2D5** immunoprecipitated both HeLa cell lysate-derived 14-3-3 eta (Lane 5) and human recombinant 14-3-3 eta (Lane 6).

Similar analyses were performed for several other hybridoma clones (data not shown). These experiments demonstrate that the monoclonal antibodies produced in Example 1 are capable of binding to and immunoprecipitating or "capturing" 14-3-3 eta in its native configuration, as evidenced by the immunoprecipitation of the protein from HeLa cell lysates.

### Example 8: Anti-14-4-3 antibody reduces MMP expression in mouse RA model; 14-3-3 antagonist peptide reduces MMP expression in mouse RA model

Collagen-induced arthritis is induced in Male DBA mice by injection of 100 µg of purified type II collagen emulsified in Freund's complete adjuvant at the base of the tail as described in Williams et al., PNAS, 89:9784-9788, 1992. Mice are inspected daily thereafter and mice that exhibit erythema and/or swelling in one of more limbs are assigned randomly to a treatment regimen with one or more antagonists 14-3-3 eta described herein or to a placebo treatment. Alternatively, a treatment regimen is begun on the day prior to immunization with type II collagen. Various treatment regimens are implemented, using groups of 10 mice, as follows:
(1) R-18 peptide is administered at various dosages ranging from 0.1 and 20 mg/kg (a) intraperitoneally or (b) into the synovium, twice weekly.
(2) Selected anti-14-3-3 eta antibodies obtained and purified from the hybridoma supernatants of Example 1 are administered at various dosages ranging from 0.10 to 20 mg/kg (a) intraperitoneally or (b) into the synovium, twice weekly.
(3) Placebo treatment

The arthritis is monitored over a 20-day treatment period, and the following disease indices are evaluated.

*Clinical score.* Mouse limbs are assessed for swelling, erythema, joint rigidity, and paw swelling. The clinical indicia of arthritis is reduced in animals in which the treatment regimen has been efficacious, as compared to placebo controls.

*14-3-3*, *MMP-1 and*/*or MMP-3 expression in the synovium.* Synovial samples are taken at various time points, and the 14-3-3, preferably 14-3-3 gamma and/or 14-3-3 eta, and MMP-1 and /or MMP-3 levels are determined. The levels of MMP-1 and MMP-3 are reduced in animals in which the treatment regimen has been efficacious, as compared to placebo controls.

*Histopathological assessment.* Arthritic paws are fixed, embedded in paraffin, sectioned and stained with hematoxylin and eosin for microscopic evaluation. The severity of arthritis in each joint is graded according to the following criteria: mild = minimal synovitis, cartilage loss, and bone erosions limited to discrete foci; moderate = synovitis and erosions present but normal joint architecture intact; severe = synovitis, extensive erosions, and joint architecture disrupted. The severity of arthritis detected by histopathology is reduced in animals in which the treatment regimen has been efficacious, as compared to placebo controls.

### Example 9 Anti-14-4-3 antibody reduces MMP expression in rabbit RA model; 14-3-3 antagonist peptide reduces MMP expression in rabbit RA model induced by implantation of cells secreting IL-1

The 14-3-3 eta antagonists of the invention are evaluated in a rabbit model in which arthritis is induced by the implantation of 5X 10⁵ IL-1 producing cells into the knee joints of New Zealand white rabbits as described in Yao et al., Arthritis Research and Therapy 2006, 8:R16, available on line at http://arthritis-research.com/content/8/1/R16. Testing and evaluation is done essentially as described in Example 8.

### Example 10 Anti-14-4-3 antibody reduces MMP expression in RA model; 14-3-3 antagonist peptide reduces MMP expression in RA

Experimental arthritis is induced in Brown Norway rats or in New Zealand white rabbits by the injection of recombinant 14-3-3 eta protein into the synovium of leg joints. Testing and evaluation is done essentially as described in Example 8.

Other models of rheumatoid arthritis (collagen-induced arthritis, "CIA") and experimental designs useful for the methods of the invention can be found for example, in the following references: Williams, Methods Mol Med. 2004;98:207-16. Collagen-induced arthritis as a model for rheumatoid arthritis; Brand, Com. Med., 55:114-122, 2005; Vierboom et al., Drug Discovery Today, 12:327-335, 2007; Sakaguchi et al., Curr. Opin. Immunol., 17:589-594,2005.

Prior to commencing an initial therapeutic regimen in a particular animal model, it is preferable to first validate the model as an inflammatory disorder model involving 14-3-3. Preferably, the levels of 14-3-3 and MMP, preferably 14-3-3 eta and/or 14-3-3 gamma, and preferably MMP-1 and/or MMP-3, are determined to show elevation following the induction of experimental arthritis in the model.

### General Methods

### Western blotting

Samples (synovial fluid or serum (2 µl of each), recombinant human 14-3-3 eta, cell lysates or cell-lysate immunoprecipitates) were subjected to SDS-PAGE analysis with 12-15% (wt/vol) acrylamide gel, and electrotransferred onto PVDF membranes. Non-specific proteins on membranes were blocked in 5% skim milk powder in PBS-0.1% Tween-20 overnight. Immunoblotting for Example 3 was performed using 2 µg/ml of 7 isoforms specific rabbit antihuman 14-3-3 polyclonal antibodies (Martin H, Patel Y, Jones D, Howell S, Robinson K and Aitken A 1993. Antibodies against the major brain isoforms of 14-3-3 protein. An antibody specific for the N-acetylated amino-terminus of a protein. FEBS Letters. 331:296-303). In some experiments, mainly Example 7, the antibodies from the hybridoma clones in Example 1 were used for the immunoprecipitation or 'capture' experiments. The immunprecipitates were resolved by SDS-PAGE and the membranes were blocked in skim milk and then incubated with primary 14-3-3 eta (1:1000, BioMol International SE-486) and then the appropriate secondary horseradish peroxidise conjugated anti-rabbit IgG or anti-mouse IgG antibodies (1:2500 dilution). Immunoreactive proteins were then visualized using the ECL plus western blotting detection system. Keratinocyte cell lysate (K), recombinant protein and /or HeLa cell lysate was used as a positive control. SF: synovial fluid; PS: patient serum.

### Patient samples

Synovial fluid was obtained from the knee joints of patients with active synovitis prior to the institution of anti-TNF therapeutics. All patients had a DAS score >6.0. Matched blood samples were obtained by standard venipuncture procedures. The clot was removed by centrifugation.

### Recombinant 14-3-3 eta

cDNA for keratinocyte-derived 14-3-3 eta was prepared from total RNA extracted from human keratinocytes, cloned and expressed in E. coli, and affinity purified, following the methods described in Ghahary et al 2004 J Invest Dermatol 122:1188-1197 (REF 36, infra). Primers used for PCR amplification of the 14-3-3 eta cDNA were (GCGAATTCCTGCAGCGGGCGCGGCTGGCCGA) (SEQ ID NO:80) and (GCTCGAGCCTGAAGGATCTTCAGTTGCCTTC) (SEQ ID NO:81).

### Untagged Recombinant 14-3-3 proteins

cDNA was derived from a human source, cloned and expressed in E.coli, and affinity purified. Primers used for the PCR amplification of the 14-3-3 eta cDNA were: (agaattcagttgccttctcctgctt) (SEQ ID NO: 82) and (acatatgggggaccggga) (SEQ ID NO:83); for 14-3-3 gamma (agaattcttaattgttgccttcgccg) (SEQ ID NO:84) and (acatatggtggaccgcgagc) (SEQ ID NO:85); for 14-3-3 beta (acatatgacaatggataaaagtgagctg) (SEQ ID NO: 86) and (agaattcttagttctctccctccccagc) (SEQ ID NO:87); for 14-3-3 epsilon (acatatggatgatcgagaggatctg) (SEQ ID NO:88) and (agaattctcactgattttcgtcttccac) (SEQ ID NO:89); for 14-3-3 sigma (acatatggagagagccagtctgatcc) (SEQ ID NO:90) and (agaattcagctctggggctcctg) (SEQ ID NO:91); for 14-3-3 theta (acatatggagaagactgagctgatcc) (SEQ ID NO:92) and (agaattcttagttttcagccccttctgc) (SEQ ID NO:93); for 14-3-3 zeta (acatatggataaaaatgagctggttc) (SEQ ID NO:94) and (agaattcttaattttcccctccttctcct) (SEQ ID NO:95).

### ELISA assay conditions

*For screening and testing:* For screening and testing, 1.0 µg/well of anti-AUG1-CLDK, anti-AUG2-KKLE, anti-AUG3-CKNS or anti-14-3-3 ETA antigen was coated onto ELISA plates in dH₂O at 50µL/well and dried down overnight at 37°C. Testing on 14-3-3 ETA antigen 0.25ug/well was coated in carbonate coating buffer and incubated at 4°C overnight.

*For testing by antibody trapping assay:* 1/10000 Goat anti-mouse IgG/IgM trapping antibody (Pierce cat# 31182) was coated onto ELISA plate in carbonate coating buffer (pH 9.6) at 100µL/well incubated overnight at 4°C.

*For testing on negative control antigen:* 0.5µg/well HT (human transferrin) antigen was coated onto ELISA plate in dH₂O at 50µL/well and dried down overnight at 37°C.

*For testing by Capture ELISA:* ELISA plate was coated with neat overgrown TC sup at 100µL/well incubated overnight at 4°C. Biotin labelled 14-3-3 ETA (or one of the six other 14-3-3 family members) was titrated from 1/500 to 1/16000 overtop and incubated for 1 hour at room temperature.

*Blocking:* Plates were blocked with 3% skim milk powder in PBS (pH 7.4) at 100µL/well and incubated for 1 hour at room temperature.

*1° antibody:* Mouse anti-AUG1-CLDK, anti-AUG2-KKLE, anti-AUG3-CKNS or anti-14-3-3 eta hybridoma tissue culture supernatant and mouse monoclonal controls were added at 100µL neat per well for screening and testing. Mouse anti- AUG1-CLDK, anti-AUG2-KKLE, anti-AUG3-CKNS or anti-14-3-3 eta immune serum and mouse pre-immune serum were diluted 1/500 in SP2/0 tissue culture supernatant added at 100µL/well for screening and testing. Incubated for 1 hour at 37°C with shaking for both the screening and testing.

2° *antibody used for screening and testing:* 1/25000 Goat anti-mouse IgG Fc HRP conjugated (Jackson cat#115-035-164) was used in screening and testing. Secondary antibody diluted in PBS-Tween added at 100µL/well and incubated for 1 hour at 37°C with shaking.

*Streptavidin used for Capture ELISA:* Add 100ul/well of Streptavidin HRPO (1:8000, CedarLane cat#CLCSA1007) and incubated for 1 hour at room temperature with shaking.

*Substrate:* TMB buffer (BioFx cat# TMBW-1000-01) was added at 50µL per well and incubated in the dark at room temperature. Reactions for screening and testing were stopped with 50µL 1 M HCl per well after 10 minutes and read at OD₄₅₀nm.

### Dot Blot Conditions:

*For Screening:* Millipore, Immobilon Transfer Membrane cat#IPVH304F0 was used. 14-3-3 ETA antigen was boiled in sample buffer 5 minutes and allowed to cool. Antigen was dotted on for a total of 6ug dot amounts with a pipettor. After allowing antigen to dry for 15 minutes blots were washed with several changes of PBS-Tween pH7.4. Blots were kept in separate petri dishes for entire screening process.
*Blocking:* The PVDF membrane was blocked with 5% milk powder in PBS (pH 7.4) for 1 hour at room temperature. Blot was washed after blocking for 15 minutes with several changes of PBS-Tween pH7.4. Blots were allowed to dry on paper towels face up for 10 minutes prior to primary antibody application.
*1° antibody:* Mouse AUG1-CLDK, anti-AUG2-KKLE, anti-AUG3-CKNS or anti-14-3-3 eta hybridoma tissue culture supernatant and mouse monoclonal controls were incubated with blots in separate petri dishes. Mouse anti-AUG1-CLDK, anti-AUG2-KKLE, anti-AUG3-CKNS or anti-14-3-3 eta immune and mouse pre-immune sera were diluted 1/500 in SP2/0 tissue culture supernatant used as controls. Blots were incubated with shaking for 1 hour at room temp. Blots were washed after primary antibody incubation for 30 minutes with 5 changes of PBS-Tween pH7.4.
*2° antibody:* 1/5000 Goat anti-mouse IgG/IgM, (H+L), Alkaline Phosphatase Conjugated (Rockland 610-4502) diluted in PBS-Tween pH 7.4 was added to the blots and incubated with shaking in Petri dishes for one hour at room temperature. Blots were washed after secondary antibody incubation for 30 minutes with 5 changes of PBS-Tween pH7.4. Blots were equilibrated in Tris 0.1 M pH 9 buffer for 10 minutes at room temp and then dripped dried before addition of substrate.

*Substrate:* BCIP/NBT developer 1 component AP membrane substrate (BioFX product # BCID-1000-01) was dripped onto blot neat at room temp. The reaction was stopped after 5 minutes with cold tap water and results were determined quantitatively by eye and given a score of strong positive +++, moderate positive ++, weak positive +, slight positive +/-, negative -.

### References

1. Harris ED Jr., History and Epidemiology of Rheumatoid Arthritis: How long has it affected us, and who is at risk? In: Rheumatoid Arthritis. Philadelphia: W.B. Saunders Company, 1997: 21-27.
2. Harris ED Jr., Introduction. In: Rheumatoid Arthritis. Philadelphia: W.B. Saunders Company, 1997: xix-xxiii.
3. Harris ED Jr., Rheumatoid Synovium: Complex, and More Than the Sum of its Parts. In: Rheumatoid Arthritis. Philadelphia: W.B. Saunders Company, 1997: 127-149.
5. Firestein GS. (1997). Rheumatoid synovitis and pannus. In: J.H. Klippel and P.A. Dieppe, Editors, Rheumatology, Mosby, London , pp. 5/13.1-5/13.5, 1997.
6. Pap T, Shigeyama Y, Kuchen S. (2000). Arthritis Rheum. 43: 1226-1232.
7. Tolboom TCA, Pieterman E, van der Laan WE. Ann. Rheum. Dis. 61: 975-980, 2002.
8. Sorsa T, Konttinen YT, Lindy O. Arthritis Rheum. 22: 44-53, 1992.
9. Lindy O, Konttinen YT, Sorsa T. Arthritis Rheum. 40:1391-1399, 1997.
10. Ahrens D, Koch AE, Pope RM. Arthritis Rheum. 39:1576-1587, 1996.
11. Smeets TJM, Dayer JM, Karan MC. Arthritis Rheum. 43:270-274, 2000.
12. Poole AR; Cartilage in health and disease. In: Koopman WJ. Ed. Arthritis and Allied conditions. A textbook of rheumatology. 14th ed. Baltimore: Williams and Wilikins, 2001: 226-284.
13. Konttinen YT, Ceponis A, Takagi M, Ainola M, Sorsa T, Sutinen M, et al. Matrix Biol. 17:585-601, 1998.
14. Katrib.A, McNeil HP, Youssef PP: Inflamm. Res. 51: 170-175, 2002.
15. Harris ED Jr., Cytokines, Lymphokines, Growth Factors, and Chemokines. In: Rheumatoid Arthritis. Philadelphia: W.B. Saunders Company, 1997: 105-125.
16. Jasser, M.Z., Mitchell P.G. and Cheung, H.S.: induction of stomelysin-1 and collagenases synthesis in fibrochondrocytes by TNF-alpha. Matrix Biology 14: 241, 1994.
17. Burger D, Rezzonico R, Li JM, Modoux C, Pierce RA, Welgus HG, Dayer JM. Arthritis Rheum. 41(10):1748-59,1998
18. Y. Yamamura, R. Gupta, Y. Morit, X. He, R. Pai, J. Endres, A. Freiberg, K. Chung and D.A. Fox. J. Immunol. 166 (2001), pp. 2270-2275
19. Miranda-Carus ME, Balsa A, Benito-Miguel M, Perez de Ayala C, Martin-Mola E. J. Immunol. 173:1463-1476, 2004
20. Cho ML, Yoon CH, Hwang CY. Arthritis Rheum. 50:776-784, 2004
21. Bombara MP, Webb DL, Conrad P. J. Leukocyte Biol. 54: 399-406, 1993.
22. McInnes IB, Leung BP, Liew FY. Arthritis Res. 2(5):374-8.34, 2000.
23. FU H, Subramanian RR, Masters SC:Annu Rev Pharmacol Toxicol 40:617-647, 2000.
24. Hsich G, Kenney K, Gibbs CJ, Lee KH, Harrington MG: N Engl J Med 335:924-30, 1996
25. Wilker E, Yaffe MB: J Mol Cell Cardiol 37: 633-642, 2004.
26. Moore et al. 1967.
27. Ichimura T, Isobe T, Okuyama T, Yamauchi T, Fujisawa H (1987) FEBS Lett. 219:79-82.
28. Ichimura T, Isobe T, Okuyama T, Takahashi N, Araki K, Kuwano R, Akahashi Y (1988).. Proc Natl Acad Sci USA, 85:7084-8.
29. Toker A, Ellis CA, Sellers LA, Atiken A 1990. Eur J Biochem 191:421-429.
30. Craparo A, Freund R, Gustafson T (1997). J Biol Chem 272:11663-69.
31. Yaffe MB (2002). FEBS Lett 513(1):53-57.
32. Hermeking H, Lengauer C, Polyak K, He TC, Zhang L, Thiagalingam S, Kinzler KW, Volgelstein B. Mol Cell 1:3-11, 1997.
33. Chan TA, Hermeking H, Lengauer C, Kinzler KW, Volgelstein B. Nature 401:616-620, 1999.
34. Laronga C, Yang HY, Neal C, Lee MH (2000). J. Biol. Chem. 275:23106-23112.
35. Wang B, Yang H, Liu Y, Jelinek T, Zhang L, Ruoslahti E, Fu H (1999) Biochemistry 38: 12499-12504.
36. Ghahary A, Karimi-Busheri F, Marcoux Y, Li Y, Tredget EE, Kilani RT, Li L, Zheng J, Karami A, Keller B, Weinfeld M. J. Invest. Dermatol. 122(5): 1188-1197,2004.

All citations are expressly incorporated herein in their entirety by reference.

### Clauses

1. A method for treating arthritis, comprising administering to a patient a therapeutically effective amount of a 14-3-3 antagonist, wherein said 14-3-3 antagonist is capable of specifically binding to an extracellularly localized 14-3-3 protein, wherein said 14-3-3 protein is selected from γ and η isoforms.
2. The method according to clause 1, wherein said 14-3-3 antagonist is a peptide.
3. The method according to clause 2, wherein said peptide comprises the amino acid sequence of R-18.
4. The method according to clause 2, wherein said peptide consists essentially of the amino acid sequence of R-18.
5. The method according to clause 2, wherein said peptide comprises a segment of the amino acid sequence of R-18.
6. The method according to clause 1, wherein said 14-3-3 antagonist is an anti-14-3-3 antibody.
7. The method according to clause 6, wherein said antibody is a monoclonal antibody.
8. The method according to clause 7, wherein said monoclonal antibody is a humanized monoclonal antibody.
9. The method according to clause 6, wherein said anti-14-3-3 antibody is an anti-14-3-3 eta antibody.
10. The method according to clause 9, wherein said anti-14-3-3 eta antibody is a pan 14-3-3 antibody.
11. The method according to clause 9, wherein said anti-14-3-3 eta antibody is capable of discriminating between 14-3-3 protein isoforms.
12. The method according to clause 9, wherein said anti-14-3-3 eta antibody is capable of specifically binding to a loop, helix, or non-helix 14-3-3 eta peptide.
13. The method according to clause 9, wherein said anti-14-3-3 eta antibody is capable of binding to an amino acid sequence selected from the group consisting of SEQ ID NOs:1-32.
14. The method according to clause 9, wherein said anti-14-3-3 eta antibody is capable of specifically binding to the amino acid sequence LDKFLIKNSNDF (SEQ ID NO:30).
15. The method according to clause 9, wherein said anti-14-3-3 eta antibody is capable of specifically binding to the amino acid sequence KKLEKVKAYR (SEQ ID NO:31).
16. The method according to clause 9, wherein said anti-14-3-3 eta antibody is capable of specifically binding to the amino acid sequence KNSVVEASEAAYKEA (SEQ ID NO:32).
17. The method according to clause 6, wherein said anti-14-3-3 antibody is an anti-14-3-3 gamma antibody.
18. The method according to clause 17, wherein said anti-14-3-3 gamma antibody is a pan 14-3-3 antibody.
19. The method according to clause 17, wherein said anti-14-3-3 gamma antibody is capable of differentiating between 14-3-3 protein isoforms.
20. The method according to clause 17, wherein said anti-14-3-3 gamma antibody is capable of specifically binding to a loop, helix, or non-helix 14-3-3 gamma peptide.
21. The method according to clause 17, wherein said anti-14-3-3 gamma antibody is capable of binding to an amino acid sequence selected from the group consisting of SEQ ID NOs:33-62.
22. An antibody for use in the method according to any one of clauses 6-21.
23. A hybridoma capable of producing the antibody according to clause 22.
24. A 14-3-3 antagonist for use in treating arthritis, wherein said 14-3-3 antagonist is capable of specifically binding to an extracellularly localized 14-3-3 protein, wherein said 14-3-3 protein is selected from γ and η isoforms.
25. The 14-3-3 antagonist of clause 24, wherein said 14-3-3 antagonist is a peptide.
26. The 14-3-3 antagonist of clause 25, wherein said peptide comprises the amino acid sequence of R-18.
27. The 14-3-3 antagonist of clause 25, wherein said peptide consists essentially of the amino acid sequence of R-18.
28. The 14-3-3 antagonist of clause 25, wherein said peptide comprises a segment of the amino acid sequence of R-18.
29. The 14-3-3 antagonist of clause 24, wherein said 14-3-3 antagonist is an anti-14-3-3 antibody.
30. The 14-3-3 antagonist of clause 29, wherein said antibody is a monoclonal antibody.
31. The 14-3-3 antagonist of clause 30, wherein said monoclonal antibody is a humanized monoclonal antibody.
32. The 14-3-3 antagonist of clause 29, wherein said anti-14-3-3 antibody is an anti-14-3-3 eta antibody.
33. The 14-3-3 antagonist of clause 32, wherein said anti-14-3-3 eta antibody is a pan 14-3-3 antibody.
34. The 14-3-3 antagonist of clause 32, wherein said anti-14-3-3 eta antibody is capable of discriminating between 14-3-3 protein isoforms.
35. The 14-3-3 antagonist of clause 32, wherein said anti-14-3-3 eta antibody is capable of specifically binding to a loop, helix, or non-helix 14-3-3 eta peptide.
36. The 14-3-3 antagonist of clause 32, wherein said anti-14-3-3 eta antibody is capable of binding to an amino acid sequence selected from the group consisting of SEQ ID NOs:1-32.
37. The 14-3-3 antagonist of clause 32, wherein said anti-14-3-3 eta antibody is capable of specifically binding to the amino acid sequence LDKFLIKNSNDF (SEQ ID NO:30).
38. The 14-3-3 antagonist of clause 32, wherein said anti-14-3-3 eta antibody is capable of specifically binding to the amino acid sequence KKLEKVKAYR (SEQ ID NO:31).
39. The 14-3-3 antagonist of clause 32, wherein said anti-14-3-3 eta antibody is capable of specifically binding to the amino acid sequence KNSVVEASEAAYKEA (SEQ ID NO:32).
40. The 14-3-3 antagonist of clause 29, wherein said anti-14-3-3 antibody is an anti-14-3-3 gamma antibody.
41. The 14-3-3 antagonist of clause 40, wherein said anti-14-3-3 gamma antibody is a pan 14-3-3 antibody.
42. The 14-3-3 antagonist of clause 40, wherein said anti-14-3-3 gamma antibody is capable of differentiating between 14-3-3 protein isoforms.
43. The 14-3-3 antagonist of clause 40, wherein said anti-14-3-3 gamma antibody is capable of specifically binding to a loop, helix, or non-helix 14-3-3 gamma peptide.
44. The 14-3-3 antagonist of clause 40, wherein said anti-14-3-3 gamma antibody is capable of binding to an amino acid sequence selected from the group consisting of SEQ ID NOs:33-62.
45. The 14-3-3 antagonist of any of clauses 29 to 44, wherein the antibody is produced by a hybridoma.
46. Use of an effective amount of a 14-3-3 antagonist for treating arthritis, wherein said 14-3-3 antagonist is capable of specifically binding to an extracellularly localized 14-3-3 protein, wherein said 14-3-3 protein is selected from γ and η isoforms.
47. Use of a 14-3-3 antagonist to formulate a medicament for treating arthritis, wherein said 14-3-3 antagonist is capable of specifically binding to an extracellularly localized 14-3-3 protein, wherein said 14-3-3 protein is selected from γ and η isoforms.
48. The use according to clause 46 or 47, wherein said 14-3-3 antagonist is a peptide.
49. The use according to clause 48, wherein said peptide comprises the amino acid sequence of R-18.
50. The use according to clause 48, wherein said peptide consists essentially of the amino acid sequence of R-18.
51. The use according to clause 48, wherein said peptide comprises a segment of the amino acid sequence of R-18.
52. The use according to clause 46 or 47, wherein said 14-3-3 antagonist is an anti-14-3-3 antibody.
53. The use according to clause 52, wherein said antibody is a monoclonal antibody.
54. The use according to clause 53, wherein said monoclonal antibody is a humanized monoclonal antibody.
55. The use according to clause 52, wherein said anti-14-3-3 antibody is an anti-14-3-3 eta antibody.
56. The use according to clause 55, wherein said anti-14-3-3 eta antibody is a pan 14-3-3 antibody.
57. The use according to clause 55, wherein said anti-14-3-3 eta antibody is capable of discriminating between 14-3-3 protein isoforms.
58. The use according to clause 55, wherein said anti-14-3-3 eta antibody is capable of specifically binding to a loop, helix, or non-helix 14-3-3 eta peptide.
59. The use according to clause 55, wherein said anti-14-3-3 eta antibody is capable of binding to an amino acid sequence selected from the group consisting of SEQ ID NOs:1-32.
60. The use according to clause 55, wherein said anti-14-3-3 eta antibody is capable of specifically binding to the amino acid sequence LDKFLIKNSNDF (SEQ ID NO:30).
61. The use according to clause 55, wherein said anti-14-3-3 eta antibody is capable of specifically binding to the amino acid sequence KKLEKVKAYR (SEQ ID NO:31).
62. The use according to clause 55, wherein said anti-14-3-3 eta antibody is capable of specifically binding to the amino acid sequence KNSVVEASEAAYKEA (SEQ ID NO:32).
63. The use according to clause 52, wherein said anti-14-3-3 antibody is an anti-14-3-3 gamma antibody.
64. The use according to clause 63, wherein said anti-14-3-3 gamma antibody is a pan 14-3-3 antibody.
65. The use according to clause 63, wherein said anti-14-3-3 gamma antibody is capable of differentiating between 14-3-3 protein isoforms.
66. The use according to clause 63, wherein said anti-14-3-3 gamma antibody is capable of specifically binding to a loop, helix, or non-helix 14-3-3 gamma peptide.
67. The use according to clause 63, wherein said anti-14-3-3 gamma antibody is capable of binding to an amino acid sequence selected from the group consisting of SEQ ID NOs:33-62.
68. The use according to any one clauses 52 - 67, wherein the antibody is produced by a hybridoma.
69. A pharmaceutical composition comprising a 14-3-3 antagonist in a formulation that provides for engagement of extracellularly localized 14-3-3 protein by said 14-3-3 antagonist following administration.
70. The pharmaceutical composition according to clause 69, wherein said 14-3-3 antagonist is an anti-14-3-3 antibody.
71. The pharmaceutical composition according to clause 69, wherein said 14-3-3 antagonist is a peptide.
72. A method for reducing MMP expression in the synovium of a patient, comprising administering to the patient a pharmaceutical composition according to clause 69.
73. The method according to clause 72, wherein said MMP is selected from the group consisting of MMP-1, 3, 8, 9, 10, 11 and 13.
74. The method according to clause 73, wherein said MMP is MMP-1 or MMP-3.
75. A pharmaceutical composition of clause 69 for use in reducing MMP expression in the synovium of a patient.
76. The pharmaceutical composition of clause 75, wherein said MMP is selected from the group consisting of MMP-1, 3, 8, 9, 10, 11 and 13.
77. The pharmaceutical composition of clause 76, wherein said MMP is MMP-1 or MMP-3.
78. Use of a pharmaceutical composition according to clause 69 for reducing MMP expression in the synovium of a patient.
79. The use according to clause 78, wherein said MMP is selected from the group consisting of MMP-1, 3, 8, 9, 10, 11 and 13.
80. The use according to clause 79, wherein said MMP is MMP-1 or MMP-3.

## Claims

1. A 14-3-3 antagonist for use in treating arthritis, wherein said 14-3-3 antagonist is capable of specifically binding to an extracellularly localized 14-3-3 protein, wherein said 14-3-3 protein is selected from eta and gamma isoforms and wherein said 14-3-3 antagonist is an peptide comprising the amino acid sequence Pro-His-Cys-Val-Pro-Arg-Asp-Leu-Ser-Trp-Leu-Asp-Leu-Glu-Ala-Asn-Met-Cys-Leu-Pro.

2. The 14-3-3 antagonist for use according to claim 1 wherein said peptide consists of the amino acid sequence Pro-His-Cys-Val-Pro-Arg-Asp-Leu-Ser-Trp-Leu-Asp-Leu-Glu-Ala-Asn-Met-Cys-Leu-Pro.

3. The 14-3-3 antagonist for use according to claim 1 wherein said peptide comprises a segment of the amino acid sequence Pro-His-Cys-Val-Pro-Arg-Asp-Leu-Ser-Trp-Leu-Asp-Leu-Glu-Ala-Asn-Met-Cys-Leu-Pro.

4. A pharmaceutical composition comprising a 14-3-3 antagonist according to any of the claims 1 to 3 in a formulation that provides for engagement of extracellularly localized 14-3-3 protein by said 14-3-3 antagonist following administration, wherein said 14-3-3 antagonist is an anti-14-3-3 antibody.

5. A pharmaceutical composition of claim 4 for use in reducing MMP expression in the synovium of a patient.

6. The pharmaceutical composition for use of claim 5, wherein said MMP is selected from the group consisting of MMP-1, 3, 8, 9, 10, 11 and 13, preferably said MMP is MMP-1 or MMP-3.
